(19) Europäisches Patentamt
European Patent Office
Office européen des brevets

(11)  EP 2 676 683 B1

(12)  **EUROPEAN PATENT SPECIFICATION**

(45) Date of publication and mention
of the grant of the patent:
**21.09.2016   Bulletin 2016/38**

(51) Int Cl.:
*A61L 27/22* *(2006.01)*      *A61K 38/00* *(2006.01)*
*C07K 14/78* *(2006.01)*      *C07K 5/083* *(2006.01)*
*A61L 26/00* *(2006.01)*

(21) Application number: **12747857.6**

(22) Date of filing: **01.02.2012**

(86) International application number:
**PCT/JP2012/052322**

(87) International publication number:
**WO 2012/111438 (23.08.2012 Gazette 2012/34)**

(54) **AQUEOUS PROTEIN SOLUTION CONTAINING A MATERIAL FOR TISSUE REGENERATION**

WÄSSRIGE PROTEINLÖSUNG ENTHALTEND EIN MATERIAL ZUR GEWEBEREGENERATION

SOLUTION PROTÉIQUE AQUEUSE CONTENANT UN MATÉRIAU POUR LA RÉGÉNÉRATION TISSULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.02.2011   JP 2011032697**

(43) Date of publication of application:
**25.12.2013   Bulletin 2013/52**

(73) Proprietors:
• **Kyoto University
Sakyo-ku
Kyoto-shi
Kyoto 606-8501 (JP)**
• **Sanyo Chemical Industries, Ltd.
Kyoto-shi, Kyoto 605-0995 (JP)**

(72) Inventors:
• **SUZUKI Shigehiko
Kyoto-shi, Kyoto 606-8501 (JP)**
• **KAWAI Katsuya
Kyoto-shi, Kyoto 606-8501 (JP)**
• **KAWABATA Shingo
Kyoto-shi, Kyoto 605-0995 (JP)**

(74) Representative: **Webster, Jeremy Mark et al
Mewburn Ellis LLP
City Tower
40 Basinghall Street
London EC2V 5DE (GB)**

(56) References cited:
WO-A1-95/24478        JP-A- H09 509 840
JP-A- H11 502 537

• **CAPPELLO J ET AL: "In-situ self-assembling
protein polymer gel systems for administration,
delivery, and release of drugs", JOURNAL OF
CONTROLLED RELEASE, ELSEVIER,
AMSTERDAM, NL, vol. 53, no. 1-3, 30 April 1998
(1998-04-30), pages 105-117, XP004121261, ISSN:
0168-3659, DOI: 10.1016/S0168-3659(97)00243-5**
• **JOSEPH CAPPELLO ED - DAVID M WISEMAN ET
AL: "Genetically engineered protein polymers",
1 January 1997 (1997-01-01), HANDBOOK OF
BIODEGRADABLE POLYMERS, CRC PRESS,
PAGE(S) 387 - 414, XP008173680, ISBN:
978-90-5702-153-4 * page 392 - page 393,
paragraph 1; table 2 * * page 407, last paragraph
- page 409, paragraph 1 ***

Remarks:
The file contains technical information submitted after the application was filed and not included in this specification

**Description**

TECHNICAL FIELD

The present disclosure relates to a material for tissue regeneration, a protein aqueous solution containing this material, and a method for transforming this material into a gel.

BACKGROUND ART

Gauzes, swabs, and the like have been used as wound dressings that are applied to disorder or wound areas (e.g. burn wounds, donor sites, incisional wounds, and traumatic skin defects) for temporary protection. These dressings rapidly absorb exudate, but unfortunately are likely to cause bacterial infection. Additionally, in the case where the surface of wounds becomes dry, they may cause pain or bleeding when removed. One known strategy to protect the surface of wounds from dryness is to use a wound dressing with an ointment or the like. This strategy, however, is not effective enough in terms of absorption of exudate, and may keep the surface of wounds too wet.

For wounds over a wide area, coating membranes made of a synthetic material, such as silicone gauzes, silicone rubber sheets, and synthetic resin (e.g. nylon or polytetrafluoroethylene) sheets having a velour-like surface, have been developed as alternative dressings to gauzes and swabs. Unfortunately, these coating membranes made of a synthetic material poorly adhere to a wound area, have low moisture permeability, are prone to cracks, and have some other problems.

A further example is collagen sponges, which are known as materials for tissue regeneration capable of not only protecting a wound area but also promoting granulation tissue formation and epithelization (Patent Literature 1). The collagen sponges have good biocompatibility, but unfortunately are likely to cause bacterial infection and bacterial growth, and may be degraded by exudate. A further problem is that the raw material thereof is not easily available. A known strategy to solve the bacterial infection problem is to cleanse a wound area with an antimicrobial agent before applying such a coating membrane made of a material of biological origin.

JP H09 509840 A and JA H11 502537 A disclose adherent adhesive compositions comprising SELP polymers and cross-linking agent.

CITATION LIST

Patent Literature

Patent Literature 1: JP-A H10-080438

SUMMARY OF INVENTION

Technical Problem

The use of an antimicrobial agent to a wound area, however, may damage healthy tissue and retard granulation tissue formation and epithelization.

An object of the present invention is to provide a material for tissue regeneration which prevents bacterial growth and promotes granulation tissue formation or epithelization.

Solution to Problem

A material for tissue regeneration of the present invention as defined in the claims contains a synthetic protein polymer (A). The synthetic protein polymer (A) contains at least one polypeptide chain (Y) and/or at least one polypeptide chain (Y'), and has a hydrophobic scale of 0.2 to 1.2, the total number of the polypeptide chains (Y) and (Y') in the synthetic protein polymer (A) is 1 to 100, the polypeptide chain (Y) consists of 2 to 200 consecutive units of one amino acid sequence (X) selected from VPGVG (2), GVGVP (3), GPP, GAP, and GAHGPAGPK (4), and the polypeptide chain (Y') is a polypeptide chain wherein 1 to 100 amino acid residues in the polypeptide chain (Y) are independently replaced with a lysine or arginine residue.

Advantageous Effects of Invention

The material for tissue regeneration used in the present invention is effective in preventing microbial growth, and very effective in promoting granulation tissue formation or epithelization.

BRIEF DESCRIPTION OF DRAWINGS

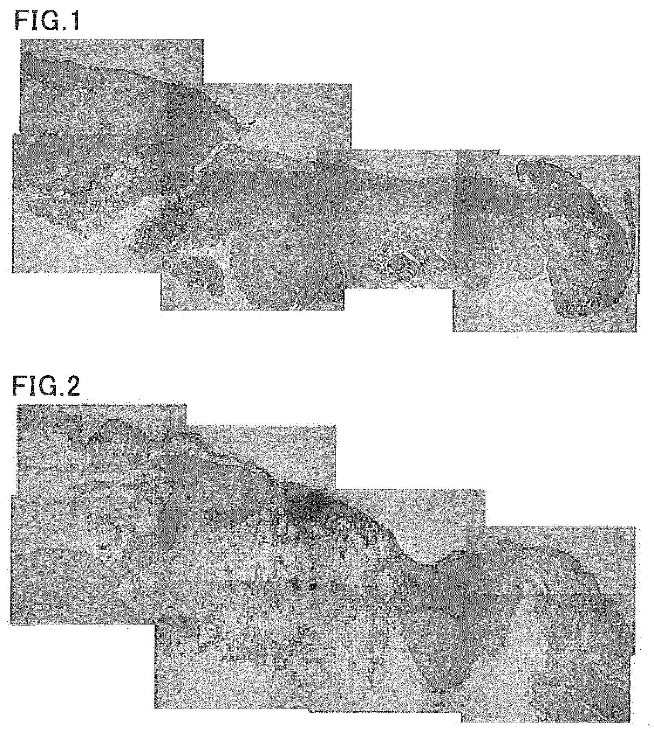

Fig. 1 is a photograph of a section of a pathological tissue specimen (HE stain) treated using a material for tissue regeneration of Example 1 in Evaluation 1; and
Fig. 2 is a photograph of a section of a pathological tissue specimen (HE stain) treated using a material for tissue regeneration of Comparative Example 1 in Evaluation 1.

DESCRIPTION OF EMBODIMENTS

The synthetic protein polymer (A) herein is a synthetic polymer that allows for elimination of components of animal origin, and can be synthesized by organic synthesis (e.g. enzymatic synthesis, solid-phase peptide synthesis, and liquid-phase synthesis), genetic recombination, and the like. Examples of the organic synthesis include methods described in "Seikagaku Jikken Koza 1 (Biochemistry Experimental Course 1), Chemistry of protein IV (edited by Japan Society for Biochemistry, published by Tokyo Kagakudojin on July 1, 1981)" or "Zoku Seikagaku Jikken Koza 2) (Sequel to Biochemistry Experimental Course 2, Chemistry of protein (Vol. 2) (edited by Japan Society for Biochemistry, published by Tokyo Kagakudojin on May 20, 1987)". Examples of the genetic recombination include the method disclosed in Japanese Patent No. 3338441. Thus, the synthetic protein polymer (A) can be produced by either the organic synthesis or the genetic recombination, but the genetic recombination, which allows for easy alteration of amino acid sequences and low-cost mass production, is preferable.

The synthetic protein polymer (A) herein contains at least one polypeptide chain (Y) and/or at least one polypeptide chain (Y'), and has a hydrophobic scale of 0.2 to 1.2, the total number of the polypeptide chains (Y) and (Y') in the synthetic protein polymer (A) is 1 to 100, the polypeptide chain (Y) consists of 2 to 200 consecutive units of one amino acid sequence (X) selected from VPGVG (2), GVGVP (3), GPP, GAP, and GAHGPAGPK (4), and the polypeptide chain (Y') is a polypeptide chain wherein 1 to 100 amino acid residues in the polypeptide chain (Y) are independently replaced with a lysine or arginine residue.

The synthetic protein polymer (A) should contain at least one amino acid sequence (X) selected from VPGVG (2), GVGVP (3), GPP, GAP, and GAHGPAGPK (4), and may contain two or more amino acid sequences (X) selected from VPGVG (2), GVGVP (3), GPP, GAP, and GAHGPAGPK (4). In the case where the synthetic protein polymer (A) contains two or more amino acid sequences (X), polypeptide chains each of which consists of 2 to 200 consecutive units of an amino acid sequence (X) are regarded as polypeptide chains (Y). In the case of two or more amino acid sequences (X) being contained in the protein polymer (A), the synthetic protein polymer (A) specifically contains, as polypeptide chains (Y), at least two sequences selected from $(VPGVG)_b$, $(GVGVP)_c$, $(GPP)_d$, $(GAP)_e$, and $(GAHGPAGPK)_f$ (b to f are each the number of consecutive units of the amino acid sequence (X), and are specifically an integer of 2 to 200).

Preferably, for cellular compatibility, the amino acid sequence (X) is VPGVG (2) or GVGVP (3). Namely, for cellular compatibility, the protein polymer (A) preferably contains a polypeptide chain (Y) represented by $(VPGVG)_b$ or a polypeptide chain (Y) represented by $(GVGVP)_c$.

In the case where two or more amino acid sequences (X) are selected from VPGVG (2), GVGVP (3), GPP, GAP, and GAHGPAGPK (4), two or more sequences selected from GPP, GVGVP (3), and GAHGPAGPK (4) are preferable for cellular compatibility, and in particular, GVGVP (3) and GAHGPAGPK (4) are preferable.

In the case where the synthetic protein polymer (A) contains two or more polypeptide chains (Y) of the same amino acid sequence (X), the numbers of consecutive units of the amino acid sequence (X) in the respective chains (Y) may be the same or different. Namely, in this case, the protein polymer (A) may contain polypeptide chains (Y), the numbers of units of which, represented by any of b to f, are the same, or polypeptide chains (Y), the numbers of consecutive units of the amino acid sequence (X) of which, represented by any of b to f, are different.

The polypeptide chain (Y) has a sequence consisting of 2 to 200 consecutive units of the amino acid sequence (X) (b to f defined above are 2 to 200). Preferably, for promotion of granulation tissue formation, the number of consecutive units is 2 to 50 (b to f defined above are 2 to 50), and more preferably 2 to 30 (b to f defined above are 2 to 30).

In the present invention, the protein polymer (A) may contain an amino acid sequence (X') wherein 1 to 5 amino acid residues in the amino acid sequence (X) are independently replaced with a lysine or arginine residue. Preferably, for hydrophilicity (solubility to water) of the amino acid sequence (X'), the number of replaced amino acid residues (amino acid residues independently replaced with a lysine or arginine residue) in the amino acid sequence (X) is 1 to 4, and more preferably 1 to 3.

Preferably, for hydrophilicity, the amino acid sequence (X') is at least one sequence selected from GKGVP (13), GKGKP (14), GKGRP (15), and GRGRP (16), and more preferably GKGVP (13), and GKGKP (14).

The polypeptide chain (Y') is a polypeptide chain wherein unit(s) of the amino acid sequence (X) of the polypeptide chain (Y) is replaced with unit(s) of the amino acid sequence (X'), and therefore 1 to 100 amino acid residues in the polypeptide chain (Y) are independently replaced with a K (lysine) or R (arginine) residue. Whether a chain can be regarded as the polypeptide chain (Y') is determined as follows: replacing all K and R residues of the synthetic protein polymer (A) with other amino acid residues (G, A, V, P, or H); and checking whether the chain of the resulting polymer coincides with the polypeptide chain (Y).

Preferably, for solubility of the polypeptide chain (Y') to water, the number of replaced amino acid in the polypeptide chain (Y) is 1 to 70, and more preferably 1 to 30.

In the case where the synthetic protein polymer (A) contains polypeptide chains (Y) the amino acid sequence (X) and/or the number of consecutive units of which is/are different, each chain is counted as one. Namely, the number of polypeptide chains (Y) is the count of the chains. The same applies to the polypeptide chain (Y').

The number of polypeptide chain(s) (Y) and/or polypeptide chain(s) (Y') is 1 to 100 in the synthetic protein polymer (A) molecule. Preferably, for promotion of granulation tissue formation, the number of the polypeptide chain(s) (Y) and/or the polypeptide chain(s) (Y') is 1 to 80, and further preferably 1 to 60.

In the present invention, the hydrophobic scale of the synthetic protein polymer (A) is 0.2 to 1.2. Preferably, for solubility and gelling properties of the synthetic protein polymer (A), the hydrophobic scale is 0.3 to 1.1, more preferably 0.4 to 1.0, and further preferably 0.5 to 1.0.

The hydrophobic scale of the synthetic protein polymer (A), which is a measure of the hydrophobicity of the synthetic protein polymer (A) molecule, is calculated by substituting the numbers ($M_\alpha$) of the respective amino acid residues in the synthetic protein polymer (A) molecule, the hydrophobic scales ($N_\alpha$) of the respective amino acid residues, and the total number of the amino acid residues in the synthetic protein polymer (A) molecule into the following equation. The hydrophobic scales of the respective amino acid residues shown below are cited from Non Patent Literature (Lehninger, Principles of Biochemistry, First volume, p.346-347).

$$\text{Hydrophobic scale} = \Sigma (M_\alpha \times N_\alpha) / (M_T)$$

$M_\alpha$: the number of residues of each amino acid in the synthetic protein polymer (A) molecule
$N_\alpha$: the hydrophobic scale of each amino acid
$M_T$: the total number of amino acid residues in the synthetic protein polymer (A) molecule

A    (alanine): 1.8
R    (arginine): -4.5
N    (asparagine): -3.5
D    (aspartic acid): -3.5
C    (cysteine): 2.5
Q    (glutamine): -3.5
E    (glutamic acid): -3.5
G    (glycine): -0.4
H    (histidine): -3.2
I    (isoleucine): 4.5
L    (leucine):3.8
K    (lysine): -3.9
M    (methionine): 1.9
F    (phenylalanine): 2.8
P    (proline): -1.6
S    (serine): -0.8
T    (threonine): -0.7
W    (tryptophan): -0.9
Y    (tyrosine): -1.3
V    (valine): 4.2

For example, in the case where the synthetic protein polymer (A) has a sequence represented by $(GVGVP)_4GKGVP(GVGVP)_3$ (8), the hydrophobic scale of the synthetic protein polymer (A) is calculated as follows:

$$\text{hydrophobic scale} = \{16 \text{ (number of G residues)} \times (-0.4) + 15 \text{ (number of V residues)} \times 4.2 + 8 \text{ (number of P residues)} \times (-1.6) + 1 \text{ (number of K residues)} \times (-3.9)\}/40 \text{ (total number of amino acid residues)} = 1.00.$$

The polypeptide chain(s) (Y) and/or polypeptide chain(s) (Y') in the synthetic protein polymer (A) attribute to high cellular compatibility and the effect of promoting granulation tissue formation and epithelization of the material for tissue regeneration of the present invention. As long as the synthetic protein polymer (A) contains the polypeptide chain(s) (Y) and/or the polypeptide chain(s) (Y'), and has a hydrophobic scale in the above range, the material for tissue regeneration is soluble to water, and the resulting aqueous solution can gel. Additionally, this aqueous solution, when used in the gel form as a material for tissue regeneration, can seal a wound area and prevent bacterial growth.

In the present invention, the synthetic protein polymer (A) may further contain the sequence GAGAGS (1). In the case where the synthetic protein polymer (A) contains the sequence GAGAGS (1), the synthetic protein polymer (A) preferably contains a polypeptide chain (S) consisting of 2 to 100 consecutive units of GAGAGS (1) for gelling properties of the synthetic protein polymer (A).

Preferably, for gelling properties, the number of consecutive units of the sequence (1) in the polypeptide chain (S) is 2 to 100, more preferably 2 to 50, still more preferably 3 to 40, and particularly preferably 4 to 30.

In the case where the synthetic protein polymer (A) contains polypeptide chain(s) (S), the number of polypeptide chain(s) (S) may be 1 in the synthetic protein polymer (A) molecule, and is preferably 1 to 20, and more preferably 3 to 10 for gelling properties.

In the case where the total number of the polypeptide chain(s) (Y), polypeptide chain(s) (Y'), and polypeptide chain(s) (S) is 2 or more, the synthetic protein polymer (A) may contain an intervening amino acid sequence (Z) between these polypeptide chains. The intervening amino acid sequence (Z) is a peptide sequence consisting of one amino acid residue, a single unit of an amino acid sequence (X), or two or more amino acid residues. Preferably, for cellular compatibility, the number of amino acid residues in the intervening amino acid sequence (Z) is 1 to 30, more preferably 1 to 15, and particularly preferably 1 to 10. Specific examples of the intervening amino acid sequence (Z) include VAAGY (5), GAAGY (6), and LGP.

The synthetic protein polymer (A) may contain a terminal amino acid sequence (T) at the N- and/or C-terminal(s) of polypeptide chain(s) (Y), polypeptide chain(s) (Y'), and/or polypeptide chain(s) (S) positioned at the ends of the synthetic protein polymer (A). The terminal amino acid sequence (T) is a peptide sequence containing one amino acid residue, a single unit of an amino acid sequence (X), or two or more amino acid residues. Preferably, for cellular compatibility, the number of amino acid residues in the terminal amino acid sequence (T) is 1 to 100, more preferably 1 to 50, and particularly preferably 1 to 40. Specific examples of the terminal amino acid sequence (T) include MDPVVLQRRDWENPGVTQLNRLAAHPPFASDPM (7).

In addition to the terminal amino acid sequence (T), the synthetic protein polymer (A) may further contain a protein or peptide having a specific amino acid sequence (hereinafter, referred to as "purification tag") at the N and/or C terminal(s) of the synthetic protein polymer (A) in order to facilitate purification or detection of expressed synthetic protein polymer (A). An affinity purification tag is used as the purification tag. Examples of the purification tag include 6xHis tag consisting of polyhistidine, V5 tag, Xpress tag, AU1 tag, T7 tag, VSV-G tag, DDDDK tag, S tag, CruzTag09™, CruzTag22™, CruzTag41™, Glu-Glu tag, Ha.11 tag, and KT3 tag.

The following shows examples of combinations of a purification tag (i) and a ligand (ii) capable of recognizing and binding to the tag.

(i-1)   Glutathione-S-transferase (GTS), (ii-1) glutathione
(i-2)   Maltose-binding protein (MBP), (ii-2) amylose
(i-3)   HQ tag, (ii-3) nickel
(i-4)   Myc tag, (ii-4) anti-Myc antibody
(i-5)   HA tag, (ii-5) anti-HA antibody
(i-6)   FLAG tag, (ii-6) anti-FLAG antibody
(i-7)   6xHis tag, (ii-7) nickel or cobalt

The purification tag sequence can be added by, for example, incorporating a nucleic acid encoding the purification tag into the 5' or 3' end of the nucleic acid encoding the synthetic protein polymer (A) in an expression vector, or using a commercial vector designed to add the purification tag.

Preferably, for promotion of granulation tissue formation, the total amount (% by weight) of units of the amino acid sequence(s) (X) and units of the amino acid sequence(s) (X') in the synthetic protein polymer (A) molecule is 10 to 80% by weight, and more preferably 15 to 75% by weight relative to the molecular mass of the synthetic protein polymer (A).

The total amount of units of the amino acid sequence(s) (X) and units of the amino acid sequence(s) (X') in the synthetic protein polymer (A) can be determined by using a protein sequencer, specifically as follows.

<How to measure amount of units of amino acid sequence (X) and units of amino acid sequence (X')>

The synthetic protein polymer (A) is divided into fragments of around 30 residues or less by two or more methods for cleaving the sequence at a specific amino acid residue. Then, the fragments were separated by high performance liquid chromatography (HPLC), and analyzed to determine their amino acid sequence using a protein sequencer. The whole sequence of the synthetic protein polymer (A) is determined by peptide mapping using the obtained amino acid sequences. Subsequently, the total amount of units of the amino acid sequence(s) (X) and units of the amino acid sequence(s) (X') is determined according to the following equation.

```
Total amount of units of amino acid sequence (X) and units
of amino acid sequence (X') (%) = [{molecular mass of amino
acid sequence (X)} × {number of units of amino acid
sequence (X)} + {molecular mass of amino acid sequence
(X')} × {number of units of amino acid sequence
(X')}]/{molecular mass of synthetic protein polymer (A)} ×
100
```

The ratio of the number of units of GAGAGS (1) and the total number of units of the amino acid sequence(s) (X) and units of amino acid sequence(s) (X') in the synthetic protein polymer (A) molecule (GAGAGS (1):amino acid sequences (X) and amino acid sequences (X') in total) is preferably (1:2) to (1:20), and more preferably (1:2) to (1:10) for gelling properties.

Preferably, for prevention of microbial growth, the molecular mass of the synthetic protein polymer (A) is 15 to 200 kDa, and more preferably 15 to 100 kDa.

The molecular mass of the synthetic protein polymer (A) can be determined by separating a sample by SDS-PAGE (SDS polyacrylamide gel electrophoresis) and comparing its migration distance with that of a standard.

In the case where the synthetic protein polymer (A) is prepared into a protein aqueous solution (B) (described later), the polypeptide chain(s) (Y) and/or polypeptide chain(s) (Y') in the protein polymer (A) contribute to the tendency of the protein aqueous solution (B) to become less flowable in response to stimulation such as heat. Specifically, when the total amount (% by weight) of units of the amino acid sequence(s) (X) and units of the amino acid sequence(s) (X') in the synthetic protein polymer (A) molecule is large, the synthetic protein polymer (A) tends to become less flowable in response to stimulation. This tendency is remarkable when the total amount of units of the sequences (X) and (X') is 50% by weight or more.

If the hydrophobic scale is too low, the synthetic protein polymer (A) has high hydrophilicity, and will not be transformed into a gel. If the hydrophobic scale is too high, the synthetic protein polymer (A) does not dissolve in water and cannot be prepared into a protein aqueous solution.

As long as the synthetic protein polymer (A) contains the polypeptide chain(s) (Y) and/or polypeptide chain(s) (Y'), and has a hydrophobic scale of 0.2 to 1.2, the synthetic protein polymer (A) can be transformed into a gel. Its ability to gel is higher when the synthetic protein polymer (A) contains GAGAGS (1). In particular, the ability is much higher when the ratio of units of GAGAGS (1) and units of the amino acid sequences (X) and (X') is (1:2) to (1:20).

When transformed into a gel in response to stimulation of heat or the like, the protein aqueous solution (B) becomes less flowable. Therefore, the protein aqueous solution (B), when applied to an affected area in this form, is less likely to spread from the affected area, and therefore is more effective in preventing microbial growth.

In the case where heat is used as the stimulation, the temperature of the later-described protein aqueous solution (B) is preferably 25 to 80°C, more preferably 25 to 50°C, particularly preferably 30 to 40°C based on a consideration of the heat stability of the synthetic protein polymer (A).

The following illustrates some preferable examples of the synthetic protein polymer (A).

(1) Synthetic protein polymer containing amino acid sequence (X) represented by GVGVP (3)

(1-1) A synthetic protein polymer (A1) containing a polypeptide chain (Y'1) wherein one amino acid residue in a polypeptide chain (Y1) consisting of consecutive units of GVGVP (3) is replaced with a K (lysine) residue

More preferred is a synthetic protein polymer (A11) containing a polypeptide chain (Y'11) represented by $(GVGVP)_4GKGVP(GVGVP)_3$ (8) and a polypeptide chain (S1-1) represented by $(GAGAGS)_4$ (9), a synthetic protein polymer (A12) containing the polypeptide chain (Y'11) and a polypeptide chain (S1-2) represented by $(GAGAGS)_2$ (10), and a synthetic protein polymer (A13) containing the polypeptide chain (Y'11), the polypeptide chain (S1-1), and the polypeptide chain (S1-2).

Specifically, there may be mentioned a synthetic protein polymer (SELP8K polymer, hydrophobic scale: 0.618, molecular mass: about 80 kDa) of sequence (11). Sequence (11) consists of 12 polypeptide chains (S1-1), 13 polypeptide chains (Y'11) and a polypeptide chain (S1-2). The polypeptide chains (S1-1) and the polypeptide chains (Y'11) are alternately linked via a chemical bond, and the polypeptide chain (S1-2) is further linked thereto via a chemical bond. The polypeptide chain (S1-1) is represented by $(GAGAGS)_4$ (9) consisting of 4 consecutive units of GAGAGS (1). The polypeptide chain (Y'11) is represented by $(GVGVP)_4GKGVP(GVGVP)_3$ (8) wherein one of V (valine) residues in the polypeptide chain (Y11) consisting of 8 consecutive units of GVGVP (3) is replaced with a K (lysine) residue. The polypeptide chain (S1-2) is represented by $(GAGAGS)_2$ sequence (10) consisting of 2 consecutive units of GAGAGS (1).

A further example is a synthetic protein polymer (SELP0K polymer, hydrophobic scale: 0.718, molecular mass: about 82 kDa) of sequence (12). Sequence (12) consists of 17 polypeptide chains (S1-2) and 17 polypeptide chains (Y'11). The polypeptide chains (S1-2) and the polypeptide chains (Y'11) are alternately linked via a chemical bond. The polypeptide chain (S1-2) is represented by $(GAGAGS)_2$ (10) consisting of 2 consecutive units of GAGAGS (1). The polypeptide chain (Y'11) is represented by $(GVGVP)_4GKGVP(GVGVP)_3$ (8).

(1-2) A synthetic protein polymer (A2) containing a polypeptide chain (Y2) consisting of consecutive units of GVGVP (3)

More preferred is a synthetic protein polymer (A21) containing a polypeptide chain (Y21) consisting of 2 consecutive units of GVGVP (3) and a polypeptide chain (S2-1) consisting of 6 consecutive units of GAGAGS (1).

Specifically, there may be mentioned a synthetic protein polymer (SLP4.1 polymer, hydrophobic scale: 0.47, molecular mass: about 93 kDa) of sequence (18). The sequence (18) contains 29 repeating amino acid blocks (L-1). The amino acid blocks (L-1) each consist of the polypeptide chain (Y21) and the polypeptide chain (S2-1). These polypeptide chains are linked via a chemical bond.

(1-3) A synthetic protein polymer (A3) containing a polypeptide chain (Y'3) wherein 2 amino acid residues in the polypeptide chain (Y1) consisting of consecutive units of GVGVP sequence (3) are replaced with K (lysine) residues

More preferred is a synthetic protein polymer (A31) containing a polypeptide chain (Y'31) consisting of two consecutive units of GKGVP (13), the polypeptide chain (S2-1) consisting of 6 units of GAGAGS (1), and a polypeptide chain (S2-2) consisting of 10 units of GAGAGS (1).

Specifically, there may be mentioned a synthetic protein polymer (SLP4.2 polymer, hydrophobic scale: 0.2, molecular mass: about 73 kDa) of sequence (26). Sequence (26) contains 10 amino acid blocks (L-2). The amino acid blocks (L-2) each consist of the polypeptide chain (S2-1) and the polypeptide chain (Y'31), and the polypeptide chain (S2-2) further linked thereto. These amino acid blocks are linked via a chemical bond.

(2) Synthetic protein polymer containing amino acid sequence (X) represented by VPGVG (2)

(2-1) A synthetic protein polymer (A4) containing a polypeptide chain (Y4) consisting of consecutive units of VPGVG (2)

Specifically, there may be mentioned a synthetic protein polymer (ELP1, hydrophobic scale: 1.2, molecular mass: about 65 kDa) of sequence (21) which contains a polypeptide chain (Y41) consisting of 160 consecutive units of VPGVG (2).

(2-2) A synthetic protein polymer (A5) containing a polypeptide chain (Y5-1) consisting of 4 consecutive units of VPGVG (2) and a polypeptide chain (Y5-2) consisting of 8 consecutive units of VPGVG (2)

More preferred is a synthetic protein polymer (A51) containing the polypeptide chain (Y5-1) consisting of 4 consecutive units of VPGVG (2), the polypeptide chain (Y5-2) consisting of 8 consecutive units of VPGVG (2), and GAGAGS (1).

Specifically, there may be mentioned a synthetic protein polymer (ELP1.1 polymer, hydrophobic scale: 1.12, molecular weight: about 220 kDa) of sequence (17). Sequence (17) contains 40 repeating amino acid blocks (L-3) each consisting of the polypeptide chain (Y5-1), GAGAGS (1), and further the polypeptide chain (Y5-2) thereto. These amino acid blocks are linked via a chemical bond.

The material for tissue regeneration used in the present invention contains the synthetic protein polymer (A). The material for tissue regeneration can be applied to an affected area, for example, by the following steps.

(1) The later-described protein aqueous solution (B) containing predetermined amounts of the material for tissue regeneration used in the present invention and water is prepared at 4 to 25°C. The aqueous solution (B) may optionally contain an inorganic salt and/or phosphoric acid (salt). The protein aqueous solution (B) may be heated immediately before applied to an affected area.
(2) The protein aqueous solution (B) is applied to the affected area.
(3) After the protein aqueous solution (B) is applied, an adequate dressing is coated thereon to prevent the protein aqueous solution (B) from spreading out from the affected area.

The amounts of the components in the protein aqueous solution (B) are as defined in the description of the protein aqueous solution (B) below, and the preferable ranges thereof are also as defined in the description below.

The preferable range of the temperature to which the protein aqueous solution (B) is heated is the same as defined in the method for transforming the synthetic protein polymer (A) of the present invention into a gel.

The aqueous solution can be applied to an affected area in any manner as long as the aqueous solution covers the affected area. Preferably, for promotion of granulation tissue formation and epithelization, the aqueous solution is applied in such a manner to fill a defective portion of the affected area.

The material of the dressing used in the step (3) is not limited at all, and examples include polyurethane, silicone, polyvinyl alcohol, polypropylene, polyester, polystyrene, polyethylene, ethylene-vinyl acetate copolymers, and nylon.

The shape of the dressing is not limited at all as long as the dressing can cover an affected area to prevent the protein aqueous solution (B) applied thereto from spreading out from the affected area. A film shape is preferable.

The material for tissue regeneration used in the present invention is free from serum of animal origin or the like, and therefore is presumed to be less antigenic. Additionally, the synthetic protein polymer (A) contains a sequence of biological origin, and therefore is presumed to be highly biocompatible. The synthetic protein polymer (A) can be mass-produced at low cost using bacteria such as E. coli, and thus the material for tissue regeneration can be easily available.

The protein aqueous solution (B) is a protein aqueous solution containing 5 to 30% by weight of the material for tissue regeneration and 70 to 95% by weight of water.

Preferably, for effectiveness in healing a wound, the amount (%by weight) of the material for tissue regeneration in the protein aqueous solution (B) is 10 to 30% by weight, and more preferably 15 to 30% by weight.

The water in the protein aqueous solution (B) is not limited at all as long as it is sterilized. Examples of sterilized water include water filtered through a microfiltration membrane having a pore diameter of not larger than 0.2 $\mu$m, water filtered through an ultrafiltration membrane, water treated with a reverse osmosis membrane, and ion exchange water sterilized by heating in an autoclave at 121°C for 20 minutes.

Preferably, for solubility of the protein polymer, the amount (% by weight) of the water in the protein aqueous solution (B) is 70 to 90% by weight, and more preferably 70 to 85% by weight relative to the weight of the protein aqueous solution (B).

The protein aqueous solution (B) may contain an inorganic salt and phosphoric acid (salt) in addition to the material for tissue regeneration and water.

Examples of the inorganic salt include sodium chloride, potassium chloride, calcium chloride, magnesium chloride, sodium sulfate, potassium sulfate, calcium sulfate, magnesium sulfate, sodium hydrogen carbonate, potassium hydrogen carbonate, calcium hydrogen carbonate, and magnesium hydrogen carbonate. The term "inorganic salt" is not intended to include phosphoric acid salts.

It is preferable that the amount (% by weight) of the salt in the protein aqueous solution (B) is equivalent to that of human biological fluids. Therefore, the amount of the salt in the protein aqueous solution (B) is preferably 0.5 to 1.3% by weight, more preferably 0.7 to 1.1% by weight, and particularly preferably 0.85 to 0.95% by weight.

The term "phosphoric acid (salt)" refers to phosphoric acid and/or phosphoric acid salts.

Examples of the phosphoric acid (salt) in the protein aqueous solution (B) include phosphoric acid and phosphoric acid salts.

Examples of the salts include alkali metal salts and alkaline-earth metal salts, more specifically, the sodium salt, potassium salt, calcium salt, and magnesium salt.

Preferably, for effectiveness in healing a wound, the amount (% by weight) of the phosphoric acid (salt) in the protein aqueous solution (B) is 0.10 to 0.30% by weight, more preferably 0.12 to 0.28% by weight, and particularly preferably 0.14 to 0.26% by weight relative to the weight of the protein aqueous solution (B).

Preferably, for tissue affinity, the pH of the protein aqueous solution (B) is 5 to 9, and more preferably 6 to 8.

Since the protein aqueous solution used in the present invention contains the material for tissue regeneration which is effective in preventing microbial growth, and also very effective in promoting granulation tissue formation and epithelization, it is also effective in promoting granulation tissue formation and epithelization at disorder or wound areas (e.g. burn wounds, donor sites, incisional wounds, and traumatic skin defects), and can be used as a dressing and filler material for a wounded area.

The method for transforming the material for tissue regeneration into a gel used in the present invention is a method for transforming the material for tissue regeneration into a gel by mixing the material for tissue regeneration and water.

The material for tissue regeneration is mixed with water into a protein aqueous solution (B). The protein aqueous solution (B) becomes less flowable and gels even at room temperature (4 to 25°C). In order to allow it to gel in a shorter time, the protein aqueous solution (B) may be heated to a temperature higher than 25°C and not higher than 80°C. At temperatures of not higher than 80°C, the protein aqueous solution gels without deteriorating the functions of the material for tissue regeneration in an adequate time period.

Preferably, for heat stability and handleability of the material for tissue regeneration, the temperature of the protein aqueous solution (B) is 4 to 80°C, more preferably 4 to 60°C, still more preferably 25 to 50°C, and particularly preferably 30 to 40°C.

EXAMPLES

The following description is offered to illustrate the present invention in more detail with reference to examples and comparative examples, and is not intended to limit the present invention.

<Preparation 1>

- Production of SELP8K polymer

Plasmid pPT0345, which encodes SELP8K, was prepared by the method disclosed in EXAMPLES of Japanese Patent No. 4088341.

The plasmid was used to transform E. coli cells into a strain capable of producing SELP8K. The following description relates to how to produce SELP8K polymer using the SELP8K-producing strain.

A culture solution of the SELP8K-producing strain which had been cultured at 30°C overnight was inoculated to 50 ml of LB medium in a 250 ml flask. Kanamycin was added at a final concentration of 50 $\mu$g per ml, and the inoculated culture solution was incubated with agitation (200 rpm) at 30°C. When the optical density of the inoculated culture solution reached an OD600 of 0.8 (measured with spectrophotometer UV1700, Shimadzu Corp.), a 40 ml aliquot was transferred to a new flask prewarmed at 42°C and incubated at the same temperature for approximately 2 hours. The culture was chilled on ice, and measured for OD600. E. coli cells were collected by centrifugation. The collected E. coli cells were lysed by sonication (4°C, 30 seconds $\times$ 10 times) to extract the protein polymer.

The protein polymer produced by the E. coli cells was separated by sodium dodecyl sulfate-polyacrylamide gel electrophoresis (SDS-PAGE), and then transferred to a polyvinylidene fluoride (PVDF) membrane. The protein was then analyzed by western blotting using a rabbit anti-SELP8K antibody and an anti-rabbit IgG-HRP labeled antibody (GE Healthcare) as a primary antibody and a secondary antibody, respectively. The apparent molecular mass of the product was found to be about 80 kDa. Thus, this analysis revealed that the SELP8K-producing strain produced a SELP8K polymer having an apparent molecular mass of 80,000 and having reactivity to the rabbit anti-SELP8K antibody.

- Purification of SELP8K polymer

The SELP8K polymer was purified from the E. coli biomass by the following steps: cell lysis; removal of insoluble cellular debris by centrifugation; and affinity chromatography. In this manner, the synthetic protein polymer having a molecular mass of about 80 kDa was recovered as a SELP8K polymer (A-1). In Example 1, the SELP8K polymer (A-1) was used as a material for tissue regeneration.

- Identification of SELP8K polymer

The obtained SELP8K polymer (A-1) was analyzed by the following procedures.

The polymer was analyzed by western blotting using the rabbit anti-SELP8K antibody and a rabbit anti-6$\times$His antibody (Roland) against a 6xHis tag at the C terminal. The band of the protein having an apparent molecular weight of 80,000 showed antibody reactivity to each antibody. Amino acid analysis of the obtained protein revealed that it was rich in glycine (43.7%), alanine (12.3%), serine (5.3%), proline (11.7%), and valine (21.2%). The product was also found to contain lysine (1.5%). Table 1 below is provided for comparison of the composition of the purified product and the predicted theoretical composition determined based on the synthetic gene sequence.

Finally, the SELP8K polymer (A-1) was found to be a synthetic protein polymer of sequence (11). As described above, sequence (11) consists of 13 polypeptide chains (Y'11), 12 polypeptide chains (S1-1), and a polypeptide chain (S1-2). The polypeptide chains (S1-1) and the polypeptide chains (Y'11) are alternately linked via a chemical bond, and the polypeptide chain (S1-2) is further linked thereto via a chemical bond. The polypeptide chain (Y'11) is represented by $(GVGVP)_4GKGVP(GVGVP)_3$ (8) wherein one of V (valine) residues in the polypeptide chain (Y11) consisting of 8 consecutive units of GVGVP (3) is replaced with a K (lysine) residue. The polypeptide chain (S1-1) is represented by $(GAGAGS)_4$ (9) consisting of 4 consecutive units of GAGAGS (1). The polypeptide chain (S1-2) is represented by $(GAGAGS)_2$ sequence (10) consisting of 2 consecutive units of GAGAGS (1).

[Table 1]

| Amino acid | Actual Ratio (%) | Theoretical Ratio (%) |
|---|---|---|
| Ala | 12.3 | 12.2 |
| Asx | 0.9 | 0.8 |
| Glx | n.d. | 0.4 |
| Phe | 0.4 | 0.1 |
| Gly | 43.7 | 41.5 |
| His | 0.4 | 0.8 |
| Ile | 0.3 | 0 |
| Lys | 1.5 | 1.5 |
| Leu | 0.3 | 0.5 |
| Met | 0.3 | 0.3 |
| Pro | 11.7 | 12.4 |
| Arg | 0.5 | 0.6 |
| Ser | 5.3 | 6.1 |
| Thr | n.d. | 0.1 |
| Val | 21.2 | 22.4 |
| Tyr | 1.1 | 0.1 |

<Preparation 2>

A synthetic protein polymer (A-2) of sequence (12) having a molecular mass of about 82 kDa was prepared in the same manner as in Preparation 1, except that "plasmid pPT0364 encoding SELP0K" was used instead of "plasmid pPT0345 encoding SELP8K".

<Preparation 3>

A synthetic protein polymer (A-3) of sequence (17) having a molecular mass of about 220 kDa was prepared in the same manner as in Preparation 1, except that "plasmid pPT0102-1 encoding ELP1.1" was used instead of "plasmid pPT0345 encoding SELP8K".

<Preparation 4>

A synthetic protein polymer (A-4) of sequence (18) having a molecular mass of about 93 kDa was prepared in the same manner as in Preparation 1, except that "pSY1398-1 encoding SLP4.1" was used instead of "plasmid pPT0345 encoding SELP8K".

<Preparation 5>

A synthetic protein polymer (A-5) of sequence (21) having a molecular mass of about 65 kDa was prepared in the same manner as in Preparation 1, except that "plasmid pPT0102 encoding ELP1" was used instead of "plasmid pPT0345 encoding SELP8K".

<Preparation 6>

A synthetic protein polymer (A-6) of sequence (23) having a molecular mass of about 73 kDa was prepared in the same manner as in Preparation 1, except that "pSY1398-2 encoding SLP4.2" was used instead of "plasmid pPT0345 encoding SELP8K".

<Preparation 7>

A synthetic protein polymer (A'-1) of sequence (19) having a molecular mass of about 70 kDa was prepared in the same manner as in Preparation 1, except that "pSY1398 encoding SLP4" was used instead of "plasmid pPT0345 encoding SELP8K".

<Preparation 8>

A synthetic protein polymer (A'-2) of sequence (20) having a molecular mass of about 66 kDa was prepared in the same manner as in Preparation 1, except that "pPT0312 encoding CLP3.7" was used instead of "plasmid pPT0345 encoding SELP8K".

<Preparation 9>

A synthetic protein polymer (A'-3) of sequence (22) having a molecular mass of about 80 kDa was prepared in the same manner as in Preparation 1, except that "plasmid pPT0161 encoding DCP2" was used instead of "plasmid pPT0345 encoding SELP8K".

<Preparation 10>

A synthetic protein polymer (A'-4) of sequence (24) having a molecular mass of about 37 kDa was prepared in the same manner as in Preparation 1, except that "plasmid pPT0102-2" encoding ELP1.2" was used instead of "plasmid pPT0345 encoding SELP8K".

<Analysis of gelling>

The synthetic protein polymers (A-1) to (A-6) and (A'-1) to (A'-4) (each 20 mg) prepared in Preparations 1 to 10 were each dissolved in 80 $\mu$L of phosphate buffer (PBS) in a plastic tube at 4°C to prepare protein aqueous solutions (B1) to (B10). These protein aqueous solutions were left standing at 37°C, and checked after 2 hours and 4 hours to make sure if gelation took place. In order to check if gelation took place, the plastic tubes containing a protein aqueous solution were felled. Solutions that did not flow out from the tubes were considered to have gelled, and solutions that flew out from the tubes were considered not to have gelled. Table 2 shows the results. In the table, "+" indicate that gelation took place, and "-" indicate that gelation did not take place.

[Table 2]

| Synthetic protein polymer | | Protein aqueous solution | Monomer Amino Acid Sequence | No. of Repeats in Polymer[2] | Hydrophobic scale | Evaluation of gelling | |
|---|---|---|---|---|---|---|---|
| | | | | | | 2 hours later | 4 hours later |
| A-1 | SELP8K | (B1) | $(GVGVP)_4GKGVP(GVGVP)_3(GAGAGS)_4$ | 13 | 0.62 | + | + |
| A-2 | SELP0K | (B2) | $(GVGVP)_4GKGVP(GVGVP)_3(GAGAGS)_2$ | 17 | 0.72 | + | + |
| A-3 | ELP1.1 | (B3) | $(VPGVG)_4GAGAGS(VPGVG)_8$ | 40 | 1.12 | - | + |
| A-4 | SLP4.1 | (B4) | $(GAGAGS)_6(GVGVP)_2$ | 29 | 0.47 | - | + |
| A-5 | ELP1 | (B5) | $(VPGVG)_4$ | 40 | 1.20 | - | + |
| A-6 | SLP4.2 | (B6) | $(GAGAGS)_6(GKGVP)_2(GAGAGS)_{10}$ | 10 | 0.2 | - | + |
| A'-1 | SLP4 | (B7) | $(GAGAGS)_6$ | 29 | 0.27 | - | - |
| A'-2 | CLP3.7 | (B8) | $(GAPGTPGPQGLPGSP)_4$ | 13 | -0.63 | - | - |
| A'-3 | DCP2 | (B9) | $(GAHGPAGPK)_2(GAPGPAGPP)_{12}(GAHGPAGPK)_2$ | 7 | -0.55 | - | - |
| A'-4 | ELP1.2 | (B10) | $(VPGVG)_4VV$ | 20 | 1.5 | - | - |

EP 2 676 683 B1

12

As seen in the results shown in Table 2, the synthetic protein polymers (A-1) to (A-6) containing polypeptide chain(s) (Y) and/or polypeptide chain(s) (Y') and having a hydrophobic scale of 0.2 to 1.2 gelled within 4 hours. In particular, the protein aqueous solution (B1) containing the synthetic protein polymer (A-1) and the protein aqueous solution (B2) containing the synthetic protein polymer (A-2) gelled within 2 hours, and thus were found to be more effective in rapidly covering/sealing a wound area.

On the other hand, the synthetic protein polymers (A'-1) and (A'-2), which were free from both the polypeptide chain (Y) and the polypeptide chain (Y'), and the synthetic protein polymers (A'-3) and (A'-4), which had a hydrophobic scale out of the range of 0.2 to 1.2, were found not to gel.

Protein aqueous solutions (B1-1) to (B1-5) and (B2-1) to (B2-4) were prepared using the synthetic protein polymers (A-1) and (A-2) prepared in Preparations 1 and 2 by adjusting the concentration of the synthetic protein polymers as shown in Table 3. The protein aqueous solutions were left standing and measured for the time until gelation took place at 25°C, 37°C, and 50°C. Table 3 shows the results.

EP 2 676 683 B1

[Table 3]

| Synthetic protein polymer | | Protein aqueous solution | Monomer Amino Acid Sequence | Concentration of material for tissue regeneration in protein aqueous solution (% by weight) | Temperature (°C) | Time until gelation (h) |
|---|---|---|---|---|---|---|
| A-1 | SELP8K | (B1-1) | $(GVGVP)_4GKGVP(GVGVP)_3(GAGAGS)_4$ | 20 | 25 | 4 |
| | | | | 20 | 37 | 2 |
| | | | | 20 | 50 | 1 |
| | | (B1-2) | | 18 | 25 | 4.5 |
| | | | | 18 | 37 | 2.5 |
| | | | | 18 | 50 | 1 |
| | | (B1-3) | | 15 | 25 | 5 |
| | | | | 15 | 37 | 3 |
| | | | | 15 | 50 | 1 |
| | | (B1-4) | | 12 | 25 | 7 |
| | | | | 12 | 37 | 4 |
| | | | | 12 | 50 | 1.5 |
| | | (B1-5) | | 10 | 25 | 8 |
| | | | | 10 | 37 | 5 |
| | | | | 10 | 50 | 2 |
| A-2 | SELP0K | (B2-1) | $(GVGVP)_4GKGVP(GVGVP)_3(GAGAGS)_2$ | 25 | 25 | 2 |
| | | | | 25 | 37 | 1.5 |
| | | | | 25 | 50 | 1 |
| | | (B2-2) | | 20 | 25 | 2.5 |
| | | | | 20 | 37 | 2 |
| | | | | 20 | 50 | 1 |
| | | (B2-3) | | 15 | 25 | 6 |
| | | | | 15 | 37 | 5 |
| | | | | 15 | 50 | 3 |

14

(continued)

| Synthetic protein polymer | | Protein aqueous solution | Monomer Amino Acid Sequence | Concentration of material for tissue regeneration in protein aqueous solution (% by weight) | Temperature (°C) | Time until gelation (h) |
|---|---|---|---|---|---|---|
| | | (B2-4) | | 10 | 25 | 18 |
| | | | | 10 | 37 | 14 |
| | | | | 10 | 50 | 5 |

The results shown in Table 3 revealed that the higher the protein concentration, the shorter the time until gelation, and the higher the heating temperature, the shorter the time until gelation.

<Example 1>

- Preparation of protein aqueous solution

A 20% by weight aqueous solution (B-1) of the SELP8K polymer (A-1) was prepared by dissolving 200 mg of the SELP8K polymer (A-1) (material for tissue regeneration) in 800 μL of phosphate buffer (PBS) at 4°C.

<Comparative Example 1>

A collagen sponge (B'-1) (Gunze Limited), which is used as artificial skin, was used.

<Evaluation 1>

(Experimental treatment on stage IV decubitus model using diabetes mouse)

Eight-week-old genetic diabetes female mice (CLEA Japan, Inc.) were anesthetized, and depilated. The skin around the third trochanter of a femur was compressed (400 g/8 mm × 2 hours × 4) to form a decubitus ulcer (diameter: 8 mm). Necrotic tissue was debrided 5 days after the compression, and 56 μl of the 20% by weight aqueous solution (B-1) of the SELP8K polymer (A-1) was applied to the debrided areas and covered with a polyurethane film. After 2 hours, the aqueous solution (B-1) had already turned into a gel. Then, the wound areas were covered with a gauze, and the gauze was fixed with SILKYTEX (ALCARE Co., Ltd.). The mice were sacrificed on the 14th day of the treatment, and skin samples were taken from the wound areas, and prepared into pathological specimens (HE stain).

Separately, pathological specimens were prepared in the same manner, except that an 8-mm diameter dressing of a collagen sponge (B'-1) was used instead of the aqueous solution (B-1), and fixed on a wound area with a nylon thread.

A set of nine pathological specimens was prepared for each of the aqueous solution (B-1) and the collagen sponge (B'-1).

The prepared pathological specimens were histologically analyzed. Specifically, the following analyses were conducted. Table 4 shows the results.

(Analysis 1) Regeneration of epidermis

Regeneration of epidermis was evaluated according to the following 5-point rating scale. The resulting ratings are the averages of the respective nine pathological specimens. A higher rating corresponds to more rapid epithelization, and thus indicates that the material for tissue regeneration was very effective in promoting epithelization.

> 1: no regenerated epidermis was observed over the entire defective portion
> 2: the area ratio of the regenerated epidermis in the entire defective portion was more than 0% and less than 25%
> 3: the area ratio of the regenerated epidermis in the entire defective portion was 25% or more and less than 50%
> 4: the area ratio of the regenerated epidermis in the entire defective portion was 50% or more and less than 75%
> 5: the area ratio of the regenerated epidermis in the entire defective portion was 75% or more

(Analysis 2) Formation of granulation tissue

Favorable granulation tissue with a combination of immature capillaries and fibroblasts was regarded as granulation tissue. Granulation tissue containing gaseous cysts or cholesterin crystals was not regarded as granulation tissue. Formation of granulation tissue was evaluated according to the following 5-point rating scale. The resulting ratings are the averages of the respective nine pathological specimens. A higher rating corresponds to more rapid granulation tissue formation, and thus indicates that the material for tissue regeneration was very effective in promoting granulation tissue formation.

> 1: no granulation tissue was observed over the entire defective portion
> 2: the area ratio of the formed tissue to the defective portion was more than 0% and less than 25%
> 3: the area ratio of the formed tissue to the defective portion was 25% or more and less than 50%
> 4: the area ratio of the formed tissue to the defective portion was 50% or more and less than 75%
> 5: the area ratio of the formed tissue to the defective portion was 75% or more

(Analysis 3) Bacterial colony

The tissue sections (HE stain) were evaluated based on the number and size of purple masses (bacterial colonies) according to the following 5-point rating scale. The resulting ratings are the averages of the respective nine pathological specimens. A lower rating corresponds to less bacterial growth, and thus indicates that the material for tissue regeneration could prevent bacterial growth.

    1: no bacterial colony was observed
    2: few small bacterial colonies were partially observed in the defective portion
    3: many or large bacterial colonies were partially observed in the defective portion
    4: many large bacterial colonies were partially observed in the defective portion
    5: bacterial colonies were observed over the entire defective portion

(Analysis 4) Gaseous cyst

The specimens were analyzed for the infection rate of aerogenic bacteria based on the distribution of gaseous cysts formed by aerogenic bacteria, and scored according to the following 5-point rating scale. The resulting ratings are the averages of the respective nine pathological specimens. A lower rating corresponds to a lower bacterial infection rate, and thus indicates that the material for tissue regeneration was resistive to bacterial infection.

    1: no gaseous cyst was observed over the entire defective portion
    2: the ratio of the gaseous cyst to the entire defective portion was higher than 0% and less than 25%
    3: the ratio of the gaseous cyst to the entire defective portion was 25% or higher and less than 50%
    4: the ratio of the gaseous cyst to the entire defective portion was 50% or higher and less than 75%
    5: the ratio of the gaseous cyst to the entire defective portion was 75% or higher

[Table 4]

|  | Material for tissue regeneration | Regeneration of epidermis | Growth of granulation tissue | Bacterial colony | Gaseous cyst |
|---|---|---|---|---|---|
| Example 1 (B-1) | SELP8K polymer | 2.3 | 3.6 | 1.2 | 2.0 |
| Comparative Example 1 (B'-1) | Collagen sponge | 1.6 | 2.7 | 2.4 | 3.2 |

<Evaluation 2>

(Microbial infection test on stage IV decubitus ulcer model using diabetes mouse)

Stage IV decubitus ulcer model mice were prepared in the same manner as in Evaluation 1, and the aqueous solution (B-1) or (B'-1) was applied to or fixed on a wound area in the same manner. The aqueous solution (B-1) was used for 7 individuals, and the collagen sponge (B'-1) was used for 6 individuals. The mice were sacrificed on the 7th day of the treatment, and skin samples were taken from the wound areas. The obtained skin samples were cut into small pieces in sterilized saline, and suspended. The suspensions (after diluted if necessary) were inoculated into nutrient agar media, and incubated at 37°C for 12 hours. The colonies on the agar plates were counted after the incubation. The result of the aqueous solution (B-1) was the average of the 7 individuals, and the result of the collagen sponge (B'-1) was the average of 6 individuals. Table 5 shows the results.

[Table 5]

|  | Material for tissue regeneration | Number of bacteria ($\times 10^6$) |
|---|---|---|
| Example 1 (B-1) | SELP8K polymer | 17 |
| Comparative Example 1 (B'-1) | Collagen sponge | 117 |

<Evaluation 3>

(Treatment test using healthy guinea pig model of full-thickness wound)

Seven-week-age healthy female guinea pigs (std: Hartley) (Japan SLC, Inc.) were anesthetized, and depilated. After cleansing, a full-thickness wound (10 × 10 mm) was formed on the back skin of each guinea pig. In the wounds, the fat layer was completely exposed. After hemostasis and drying, the 20% by weight aqueous solution (B-1) of the SELP8K polymer (A-1) was applied to the wounds, and a polyurethane film was attached thereto. Thereafter, each wound area was covered with a gauze, and the gauze was fixed to the skin around the wound areas with a nylon thread. The guinea pigs were sacrificed on the 5th or 10th day of the treatment, and skin samples were taken from the wound areas, and prepared into pathological specimens (HE stain). Specifically, 10 pathological specimens were prepared on the 5th day of the treatment, and 11 pathological specimens were prepared on the 10th day of the treatment.

Pathological specimens were prepared in the same manner, except that an 8-mm diameter dressing of the collagen sponge (B'-1) was used instead of the aqueous solution (B-1), and fixed on a wound area with a nylon thread. Specifically, 6 pathological specimens were prepared on the 5th day of the treatment, and 12 pathological specimens were prepared on the 10th day of the treatment.

The pathological specimens taken on the 5th day of the treatment were measured for the height of granulation tissue from the panniculus with a microruler. The result of the (B-1) was the average of the 10 specimens, and the result of the (B'-1) was the average of the 6 specimens. Table 6 shows the results. The pathological specimens taken on the 10th day of the treatment were measured for the length of developed epithelium from healthy tissue with a microruler. The result of the (B-1) was the average of the 11 specimens, and the result of the (B'-1) was the average of the 12 specimens. Table 7 shows the results.

[Table 6]

|  | Material for tissue regeneration | Height of granulation tissue (mm) |
|---|---|---|
| Example 1 (B-1) | SELP8K polymer | 0.64 |
| Comparative Example 1 (B'-1) | Collagen sponge | 0.44 |

[Table 7]

|  | Material for tissue regeneration | Length of developed epithelium (mm) |
|---|---|---|
| Example 1 (B-1) | SELP8K polymer | 3.90 |
| Comparative Example 1 (B'-1) | Collagen sponge | 2.23 |

As seen from the results of Evaluation 1 (Analyses 1 and 2 in Table 4) and Evaluation 3 (Tables 6 and 7), the material for tissue regeneration of Example 1 containing a polypeptide chain (Y') was more effective in promoting both granulation tissue formation and epithelization than the collagen sponge of Comparative Example 1. As seen from the results of Evaluation 1 (Analyses 3 and 4 in Table 4) and Evaluation 2 (Table 5), the material for tissue regeneration of Example 1 was remarkably effective in preventing microbial growth.

INDUSTRIAL APPLICABILITY

The material for tissue regeneration used in the present invention is effective in preventing microbial growth, and promoting granulation tissue formation and epithelization. Accordingly, it can be used not only as a dressing for covering a wound to protect disorder or wound areas (e.g. burn wounds, donor sites, incisional wounds, and traumatic skin defects), but also as a material for tissue regeneration for promoting granulation tissue formation and epithelization.

**Claims**

1. A protein aqueous solution for use in the treatment of the human or animal body by promoting granulation tissue formation or epithelization, the protein aqueous solution being in the form of a gel and comprising

   a material for tissue regeneration comprising a synthetic protein polymer (A),
   wherein the synthetic protein polymer (A) comprises at least one polypeptide chain (Y) and/or at least one polypeptide chain (Y'), and has a hydrophobic scale of 0.2 to 1.2,
   the total number of the polypeptide chains (Y) and (Y') in the synthetic protein polymer (A) is 1 to 100,
   the polypeptide chain (Y) consists of 2 to 200 consecutive units of one amino acid sequence (X) selected from VPGVG (2), GVGVP (3), GPP, GAP, and GAHGPAGPK (4)
   the polypeptide chain (Y') is a polypeptide chain wherein 1 to 100 amino acid residues in the polypeptide chain (Y) are independently replaced with a lysine or arginine residue,
   wherein the hydrophobic scale of the synthetic protein polymer (A) is defined by the following formula:

$$\text{Hydrophobic scale} = \Sigma(M_\alpha \times N_\alpha)/(M_T)$$

   wherein

   $M_\alpha$ = the number of residues of each amino acid in the synthetic protein polymer (A) molecule;
   $N_\alpha$ = the hydrophobic scale of each amino acid;
   $M_T$ = the total number of amino acid residues in the synthetic protein polymer (A) molecule;

   wherein the hydrophobic scales of the respective amino acid residues are as follows:

   A (alanine): 1.8
   R (arginine): -4.5
   N (asparagine): -3.5
   D (aspartic acid): -3.5
   C (cysteine): 2.5
   Q (glutamine): -3.5
   E (glutamic acid): -3.5
   G (glycine): -0.4
   H (histidine): -3.2
   I (isoleucine): 4.5
   L (leucine): 3.8
   K (lysine): -3.9
   M (methionine): 1.9
   F (phenylalanine): 2.8
   P (proline): -1.6
   S (serine): -0.8
   T (threonine): -0.7
   W (tryptophan): -0.9
   Y (tyrosine): -1.3
   V (valine): 4.2.

2. The protein aqueous solution for use according to claim 1,
   wherein the synthetic protein polymer (A) further comprises a polypeptide chain (S) consisting of 2 to 50 consecutive units of GAGAGS (1).

3. The protein aqueous solution for use according to claim 2,
   wherein the ratio of the number of the units of GAGAGS (1) and the total number of the units of the amino acid

78

sequence (X) and units of an amino acid sequence (X') in the synthetic protein polymer (A) molecule is (1:2) to (1:20), and

the amino acid sequence (X') is an amino acid sequence wherein 1 to 5 amino acid residues in the amino acid sequence (X) are independently replaced with a lysine or arginine residue.

4. The protein aqueous solution for use according to any one of claims 1 to 3, wherein the synthetic protein polymer (A) has a molecular mass, as determined by SDS-PAGE (SDS polyacrylamide gel electrophoresis), of 15 to 200 kDa.

5. The protein aqueous solution for use according to any one of claims 1 to 4, wherein the synthetic protein polymer (A) is a synthetic polymer (A1) comprising a polypeptide chain (Y'1), the polypeptide chain (Y'1) is obtainable by replacing one amino acid residue in a polypeptide chain (Y1) with a lysine residue, and the amino acid sequence (X) of the polypeptide chain (Y1) is GVGVP (3).

6. The protein aqueous solution for use according to any one of claims 2 to 5, wherein the synthetic protein polymer (A) is a synthetic polymer (A11) comprising a polypeptide chain (S1-1) represented by (GAGAGS)$_4$ (9) and a polypeptide chain (Y'1) represented by (GVGVP)$_4$GKGVP(GVGVP)$_3$ (8).

7. A protein aqueous solution for use according to any one of claims 1 to 6, wherein the material for tissue regeneration is present in the protein aqueous solution in an amount of 5 to 30 % by weight; and water is present in the protein aqueous solution in an amount of 70 to 95% by weight.

8. A protein aqueous solution for use according to any one of claims 1 to 7, wherein the hydrophobic scale of the synthetic protein polymer (A) is 0.5 to 1.0.

9. A protein aqueous solution for use according to any one of claims 1 to 8, wherein the number of the polypeptide chain(s) (Y) and/or the polypeptide chain(s) (Y') is 1 to 80.

10. A protein aqueous solution for use according to any one of claims 1 to 9, wherein the material for tissue regeneration is present in the protein aqueous solution in an amount of 10 to 30% by weight.

11. The protein aqueous solution for use according to any one of claims 1 to 10 in a dressing or filler material for a wounded area.

**Patentansprüche**

1. Wässrige Proteinlösung zur Verwendung bei der Behandlung eines menschlichen oder tierischen Körpers durch Förderung der Bildung von Granulationsgewebe oder von Epithelisierung, wobei die wässrige Proteinlösung in Form eines Gels vorliegt und Folgendes umfasst:

ein Material zur Geweberegeneration, das ein synthetisches Proteinpolymer (A) umfasst, wobei das synthetische Proteinpolymer (A) zumindest eine Polypeptidkette (Y) und/oder zumindest eine Polypeptidkette (Y') umfasst und einen Hydrophobiewert von 0,2 bis 1,2 aufweist, wobei die Gesamtanzahl der Polypeptidketten (Y) und der Polypeptidketten (Y') im synthetischen Proteinpolymer (A) 1 bis 100 beträgt, wobei die Polypeptidkette (Y) aus 2 bis 200 aufeinanderfolgenden Einheiten einer Aminosäuresequenz (X), ausgewählt aus VPGVG (2), GVGVP (3), GPP, GAP und GAHGPAGPK (4) besteht, wobei die Polypeptidkette (Y') eine Polypeptidkette ist, worin 1 bis 100 Aminosäurereste in der Polypeptidkette (Y) unabhängig voneinander durch einen Lysin- oder Argininrest ersetzt sind, wobei der Hydrophobiewert des synthetischen Proteinpolymers (A) durch die folgende Formel definiert ist:

$$\text{Hydrophobiewert} = \Sigma\, (M_\alpha \times N_\alpha)\, /\, (M_T),$$

worin

$M_\alpha$ = Anzahl an Resten jeder Aminosäure im Molekül des synthetischen Proteinpolymers (A);
$N_\alpha$ = Hydrophobiewert jeder Aminosäure;
$M_T$ = Gesamtanzahl an Aminosäureresten im Molekül des synthetischen Proteinpolymers (A);

wobei die Hydrophobiewerte der jeweiligen Aminosäurereste wie folgt sind:

A (Alanin): 1,8
R (Arginin): -4,5
N (Asparagin): -3,5
D (Asparaginsäure): -3,5
C (Cystein): 2,5
Q (Glutamin): -3,5
E (Glutaminsäure): -3,5
G (Glycin): -0,4
H (Histidin): -3,2
I (Isoleucin): 4,5
L (Leucin): 3,8
K (Lysin): -3,9
M (Methionin): 1,9
F (Phenylalanin): 2,8
P (Prolin): -1,6
S (Serin): -0,8
T (Threonin): 0,7
W (Tryptophan): -0,9
Y (Tyrosin): -1,3
V (Valin): 4,2.

2. Wässrige Proteinlösung zur Verwendung nach Anspruch 1,
wobei das synthetische Proteinpolymer (A) weiters eine Polypeptidkette (S) umfasst, die aus 2 bis 50 aufeinanderfolgenden Einheiten von GAGAGS (1) besteht.

3. Wässrige Proteinlösung zur Verwendung nach Anspruch 2,
wobei das Verhältnis der Anzahl der Einheiten GAGAGS (1) zur Gesamtanzahl der Einheiten der Aminosäuresequenz (X) und der Einheiten einer Aminosäuresequenz (X') im Molekül des synthetischen Proteinpolymers (A) (1:2) bis (1:20) beträgt, und wobei
die Aminosäuresequenz (X') eine Aminosäuresequenz ist, worin 1 bis 5 Aminosäurereste in der Aminosäuresequenz (X) unabhängig voneinander durch einen Lysin- oder Argininrest ersetzt sind.

4. Wässrige Proteinlösung zur Verwendung nach einem der Ansprüche 1 bis 3,
wobei das synthetische Proteinpolymer (A) eine durch SDS-PAGE (SDS-Polyacrylamid-Gelelektrophorese) bestimmte Molekülmasse von 15 bis 200 kDa aufweist.

5. Wässrige Proteinlösung zur Verwendung nach einem der Ansprüche 1 bis 4,
wobei das synthetische Proteinpolymer (A) ein synthetisches Polymer (A1) ist, das eine Polypeptidkette (Y'1) umfasst,
wobei die Polypeptidkette (Y'1) durch Ersetzen eines Aminosäurerests in einer Polypeptidkette (Y1) durch einen Lysinrest erhältlich ist, und
wobei die Aminosäuresequenz (X) der Polypeptidkette (Y1) GVGVP (3) ist.

6. Wässrige Proteinlösung zur Verwendung nach einem der Ansprüche 2 bis 5,
wobei das synthetische Proteinpolymer (A) ein synthetisches Polymer (A11) ist, das eine Polypeptidkette (S1-1), dargestellt durch $(GAGAGS)_4$ (9), und eine Polypeptidkette (Y'1), dargestellt durch $(GVGVP)_4GKGVP(GVGVP)_3$ (8), umfasst.

7. Wässrige Proteinlösung zur Verwendung nach einem der Ansprüche 1 bis 6,
wobei das Material zur Geweberegeneration in einem Anteil von 5 bis 50 Gew.-% in der wässrigen Proteinlösung enthalten ist; und
Wasser in einem Anteil von 70 bis 95 Gew.-% in der wässrigen Proteinlösung enthalten ist.

8. Wässrige Proteinlösung zur Verwendung nach einem der Ansprüche 1 bis 7,
wobei der Hydrophobiewert des synthetischen Proteinpolymers (A) 0,5 bis 1,0 beträgt.

9. Wässrige Proteinlösung zur Verwendung nach einem der Ansprüche 1 bis 8, wobei die Anzahl der Polypeptidkette(n) (Y) und/oder der Polypeptidkette(n) (Y') 1 bis 80 beträgt.

10. Wässrige Proteinlösung zur Verwendung nach einem der Ansprüche 1 bis 9,
wobei das Material für die Geweberegeneration in einem Anteil von 10 bis 30 Gew.-% in der wässrigen Proteinlösung enthalten ist.

11. Wässrige Proteinlösung zur Verwendung nach einem der Ansprüche 1 bis 10 in einem Verband- oder Füllmaterial für einen Wundbereich.


**Revendications**

1. Solution protéique aqueuse destinée à être utilisée dans le traitement du corps humain ou animal en favorisant la formation de tissu de granulation ou l'épithélialisation, la solution protéique aqueuse étant sous la forme d'un gel et comprenant
un matériau pour la régénération tissulaire comprenant un polymère protéique synthétique (A),
où le polymère protéique synthétique (A) comprend au moins une chaîne polypeptidique (Y) et/ou au moins une chaîne polypeptidique (Y'), et possède une échelle hydrophobe de 0,2 à 1,2,
le nombre total des chaînes polypeptidiques (Y) et (Y') dans le polymère protéique synthétique (A) est de 1 à 100,
la chaîne polypeptidique (Y) consiste en 2 à 200 unités consécutives d'une séquence d'acides aminés (X) sélectionnée parmi VPGVG (2), GVGVP (3), GPP, GAP, et GAHGPAGPK (4)
la chaîne polypeptidique (Y') est une chaîne polypeptidique dans laquelle 1 à 100 résidus d'acides aminés dans la chaîne polypeptidique (Y) sont indépendamment remplacés par un résidu lysine ou arginine,
où l'échelle hydrophobe du polymère protéique synthétique (A) est définie par la formule suivante :

$$\text{Échelle hydrophobe} = \Sigma(M_\alpha \times N_\alpha)/(M_T)$$

dans laquelle

$M_\alpha$ = le nombre de résidus de chaque acide aminé dans la molécule de polymère protéique synthétique (A) ;
$N_\alpha$ = l'échelle hydrophobe de chaque acide aminé ;
$M_T$ = le nombre total de résidus d'acides aminés dans la molécule de polymère protéique synthétique (A) ;

où les échelles hydrophobes des résidus d'acides aminés respectifs sont les suivantes :

A (alanine) : 1,8
R (arginine) : -4,5
N (asparagine) : -3,5
D (acide aspartique) : -3,5
C (cystéine) : 2,5
Q (glutamine) : -3,5
E (acide glutamique) : -3,5
G (glycine) : -0,4
H (histidine) : -3,2
I (isoleucine) : 4,5
L (leucine) : 3,8
K (lysine) : -3,9
M (méthionine) : 1,9
F (phénylalanine) : 2,8

P (proline) : -1,6
S (sérine) : -0,8
T (thréonine) : -0,7
W (tryptophane) : -0,9
Y (tyrosine) : -1,3
V (valine) : 4,2.

2. Solution protéique aqueuse destinée à être utilisée selon la revendication 1,
dans laquelle le polymère protéique synthétique (A) comprend en outre une chaîne polypeptidique (S) consistant en 2 à 50 unités consécutives de GAGAGS (1).

3. Solution protéique aqueuse destinée à être utilisée selon la revendication 2,
dans laquelle le rapport du nombre des unités de GAGAGS (1) et du nombre total des unités de la séquence d'acides aminés (X) et des unités d'une séquence d'acides aminés (X') dans la molécule de polymère protéique synthétique (A) est de (1:2) à (1:20), et
la séquence d'acides aminés (X') est une séquence d'acides aminés dans laquelle 1 à 5 résidus d'acides aminés dans la séquence d'acides aminés (X) sont indépendamment remplacés par un résidu lysine ou arginine.

4. Solution protéique aqueuse destinée à être utilisée selon l'une quelconque des revendications 1 à 3,
dans laquelle le polymère protéique synthétique (A) possède une masse moléculaire, telle que déterminée par SDS-PAGE (électrophorèse sur gel de polyacrylamide-SDS), de 15 à 200 kDa.

5. Solution protéique aqueuse destinée à être utilisée selon l'une quelconque des revendications 1 à 4,
dans laquelle le polymère protéique synthétique (A) est un polymère synthétique (A1) comprenant une chaîne polypeptidique (Y'1),
la chaîne polypeptidique (Y'1) pouvant être obtenue en remplaçant un résidu d'acide aminé dans une chaîne polypeptidique (Y1) par un résidu lysine, et
la séquence d'acides aminés (X) de la chaîne polypeptidique (Y1) est GVGVP (3).

6. Solution protéique aqueuse destinée à être utilisée selon l'une quelconque des revendications 2 à 5,
dans laquelle le polymère protéique synthétique (A) est un polymère synthétique (A11) comprenant une chaîne polypeptidique (S1-1) représentée par $(GAGAGS)_4(9)$ et une chaîne polypeptidique (Y'1) représentée par $(GVGVP)_4GKGVP(GVGVP)_3(8)$.

7. Solution protéique aqueuse destinée à être utilisée selon l'une quelconque des revendications 1 à 6,
dans laquelle le matériau pour la régénération tissulaire est présent dans la solution protéique aqueuse en une quantité de 5 à 30 % en poids ; et
de l'eau est présente dans la solution protéique aqueuse en une quantité de 70 à 95 % en poids.

8. Solution protéique aqueuse destinée à être utilisée selon l'une quelconque des revendications 1 à 7,
dans laquelle l'échelle hydrophobe du polymère protéique synthétique (A) est de 0,5 à 1,0.

9. Solution protéique aqueuse destinée à être utilisée selon l'une quelconque des revendications 1 à 8,
dans laquelle le nombre de chaîne(s) polypeptidique(s) (Y) et/ou de chaîne(s) polypeptidique(s) (Y') est de 1 à 80.

10. Solution protéique aqueuse destinée à être utilisée selon l'une quelconque des revendications 1 à 9,
dans laquelle le matériau pour la régénération tissulaire est présent dans la solution protéique aqueuse en une quantité de 10 à 30 % en poids.

11. Solution protéique aqueuse destinée à être utilisée selon l'une quelconque des revendications 1 à 10 dans un pansement ou un matériau de comblement pour une surface lésée.

## FIG.1

## FIG.2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H09509840 A
- JP H11502537 A
- JP H10080438 A
- JP 3338441 B
- JP 4088341 B
- JP 2011032697 A

**Non-patent literature cited in the description**

- Seikagaku Jikken Koza 1. Tokyo Kagakudojin, 01 July 1981
- Zoku Seikagaku Jikken Koza 2. Tokyo Kagakudojin, 20 May 1987, vol. 2
- **LEHNINGER.** *Principles of Biochemistry,* 346-347

SEQUENCE LISTING

<110>        \215\221\227§\221å\212w\226@\2201\213\236\223s\221å\212w\201@KYOTO        UNIVERSITY.
\2160\227m\211»\220¬\215H\213Æ\212\224\216®\211ï\216Ð\201@SANYO CHEMICAL INDUSTRIES, LTD.

EP 2 676 683 B1

<120>                    \221g\220D\215Ä\220¶\227p\215þ\227¿\201A\202±\202ê\202ð\212Ü\202þ\203^\203
\223\203þ\203N\216¿\220\205\227n\211t\213y\202Ñ\202±\202ê\202ð\203Q\203\213
\211»\202³\202¹\202é\225û\226@

<130> 441-PCT

<150> JP2011-032697
<151> 2011-02-18

<160> 24

<170> PatentIn version 3.3

<210> 1
<211> 6
<212> PRT
<213> artificial sequence

<220>
<223> amino acid sequence

<400> 1

```
Gly Ala Gly Ala Gly Ser
1               5
```

<210> 2
<211> 5
<212> PRT
<213> Homo sapiens

<400> 2

```
Val Pro Gly Val Gly
1               5
```

<210> 3
<211> 5
<212> PRT
<213> Homo sapiens

<400> 3

```
Gly Val Gly Val Pro
1               5
```

<210> 4
<211> 9
<212> PRT
<213> Homo sapiens

<400> 4

```
Gly Ala His Gly Pro Ala Gly Pro Lys
1               5
```

<210> 5
<211> 5

<210> PRT
<213> Homo sapiens

<400> 5

```
                    Val Ala Ala Gly Tyr
                    1               5
```

<210> 6
<211> 5
<212> PRT
<213> Homo sapiens

<400> 6

```
                    Gly Ala Ala Gly Tyr
                    1               5
```

<210> 7
<211> 33
<212> PRT
<213> artificial sequence

<220>
<223> Terminal amino acid sequence

<400> 7

```
        Met Asp Pro Val Val Leu Gln Arg Arg Asp Trp Glu Asn Pro Gly Val
        1               5                   10                  15


        Thr Gln Leu Asn Arg Leu Ala Ala His Pro Pro Phe Ala Ser Asp Pro
                        20                  25                  30


        Met
```

<210> 8
<211> 40
<212> PRT
<213> artificial sequence

<220>
<223> Polypeptide chain

<400> 8

```
        Gly Val Gly Val Pro Gly Val Gly Val Pro Gly Val Gly Val Pro Gly
        1               5                   10                  15


        Val Gly Val Pro Gly Lys Gly Val Pro Gly Val Gly Val Pro Gly Val
                        20                  25                  30


        Gly Val Pro Gly Val Gly Val Pro
                    35              40
```

<210> 9
<211> 24
<212> PRT
<213> artificial sequence

<220>
<223> Polypeptide chain

<400> 9

```
Gly Ala Gly Ala Gly Ser Gly Ala Gly Ala Gly Ser Gly Ala Gly Ala
1               5                   10                  15


Gly Ser Gly Ala Gly Ala Gly Ser
            20
```

<210> 10
<211> 12
<212> PRT
<213> artificial sequence

<220>
<223> Polypeptide chain

<400> 10

```
Gly Ala Gly Ala Gly Ser Gly Ala Gly Ala Gly Ser
1               5                   10
```

<210> 11
<211> 884
<212> PRT
<213> artificial sequence

<220>
<223> \202r\202d\202k\202o\202W\202j polymer

<400> 11

```
Met Asp Pro Val Val Leu Gln Arg Arg Asp Trp Glu Asn Pro Gly Val
1               5                   10                  15


Thr Gln Leu Asn Arg Leu Ala Ala His Pro Pro Phe Ala Ser Asp Pro
            20                  25                  30


Met Gly Ala Gly Ser Gly Ala Gly Ala Gly Ser Gly Val Gly Val Pro
            35                  40                  45
```

Gly Val Gly Val Pro Gly Val Gly Val Pro Gly Val Gly Val Pro Gly
        50              55              60

Lys Gly Val Pro Gly Val Gly Val Pro Gly Val Gly Val Pro Gly Val
65              70              75              80

Gly Val Pro Gly Ala Gly Ala Gly Ser Gly Ala Gly Ala Gly Ser Gly
            85              90              95

Ala Gly Ala Gly Ser Gly Ala Gly Ala Gly Ser Gly Val Gly Val Pro
        100             105             110

Gly Val Gly Val Pro Gly Val Gly Val Pro Gly Val Gly Val Pro Gly
        115             120             125

Lys Gly Val Pro Gly Val Gly Val Pro Gly Val Gly Val Pro Gly Val
    130             135             140

Gly Val Pro Gly Ala Gly Ala Gly Ser Gly Ala Gly Ala Gly Ser Gly
145             150             155             160

Ala Gly Ala Gly Ser Gly Ala Gly Ala Gly Ser Gly Val Gly Val Pro
            165             170             175

Gly Val Gly Val Pro Gly Val Gly Val Pro Gly Val Gly Val Pro Gly
        180             185             190

Lys Gly Val Pro Gly Val Gly Val Pro Gly Val Gly Val Pro Gly Val
    195             200             205

Gly Val Pro Gly Ala Gly Ala Gly Ser Gly Ala Gly Ala Gly Ser Gly
    210             215             220

Ala Gly Ala Gly Ser Gly Ala Gly Ala Gly Ser Gly Val Gly Val Pro
225             230             235             240

Gly Val Gly Val Pro Gly Val Gly Val Pro Gly Val Gly Val Pro Gly
            245             250             255

Lys Gly Val Pro Gly Val Gly Val Pro Gly Val Gly Val Pro Gly Val
        260             265             270

Gly Val Pro Gly Ala Gly Ala Gly Ser Gly Ala Gly Ala Gly Ser Gly
        275             280             285

Ala Gly Ala Gly Ser Gly Ala Gly Ala Gly Ser Gly Val Gly Val Pro
        290             295             300

Gly Val Gly Val Pro Gly Val Gly Val Pro Gly Val Gly Val Pro Gly
305                 310                 315                 320

Lys Gly Val Pro Gly Val Gly Val Pro Gly Val Gly Val Pro Gly Val
                325                 330                 335

Gly Val Pro Gly Ala Gly Ala Gly Ser Gly Ala Gly Ala Gly Ser Gly
            340                 345                 350

Ala Gly Ala Gly Ser Gly Ala Gly Ala Gly Ser Gly Val Gly Val Pro
        355                 360                 365

Gly Val Gly Val Pro Gly Val Gly Val Pro Gly Val Gly Val Pro Gly
        370                 375                 380

Lys Gly Val Pro Gly Val Gly Val Pro Gly Val Gly Val Pro Gly Val
385                 390                 395                 400

Gly Val Pro Gly Ala Gly Ala Gly Ser Gly Ala Gly Ala Gly Ser Gly
                405                 410                 415

Ala Gly Ala Gly Ser Gly Ala Gly Ala Gly Ser Gly Val Gly Val Pro
            420                 425                 430

Gly Val Gly Val Pro Gly Val Gly Val Pro Gly Val Gly Val Pro Gly
        435                 440                 445

Lys Gly Val Pro Gly Val Gly Val Pro Gly Val Gly Val Pro Gly Val
        450                 455                 460

Gly Val Pro Gly Ala Gly Ala Gly Ser Gly Ala Gly Ala Gly Ser Gly
465                 470                 475                 480

Ala Gly Ala Gly Ser Gly Ala Gly Ala Gly Ser Gly Val Gly Val Pro
            485                 490                 495

Gly Val Gly Val Pro Gly Val Gly Val Pro Gly Val Gly Val Pro Gly
            500                 505                 510

Lys Gly Val Pro Gly Val Gly Val Pro Gly Val Gly Val Pro Gly Val
        515                 520                 525

Gly Val Pro Gly Ala Gly Ala Gly Ser Gly Ala Gly Ala Gly Ser Gly
        530                 535                 540

Ala Gly Ala Gly Ser Gly Ala Gly Ala Gly Ser Gly Val Gly Val Pro
545                 550                 555                 560

23

Gly Val Gly Val Pro Gly Val Gly Val Pro Gly Val Gly Val Pro Gly
                565                     570                     575

Lys Gly Val Pro Gly Val Gly Val Pro Gly Val Gly Val Pro Gly Val
                580                     585                     590

Gly Val Pro Gly Ala Gly Ala Gly Ser Gly Ala Gly Ala Gly Ser Gly
                595                     600                     605

Ala Gly Ala Gly Ser Gly Ala Gly Ala Gly Ser Gly Val Gly Val Pro
    610                     615                     620

Gly Val Gly Val Pro Gly Val Gly Val Pro Gly Val Gly Val Pro Gly
625                     630                     635                     640

Lys Gly Val Pro Gly Val Gly Val Pro Gly Val Gly Val Pro Gly Val
                645                     650                     655

Gly Val Pro Gly Ala Gly Ala Gly Ser Gly Ala Gly Ala Gly Ser Gly
                660                     665                     670

Ala Gly Ala Gly Ser Gly Ala Gly Ala Gly Ser Gly Val Gly Val Pro
    675                     680                     685

Gly Val Gly Val Pro Gly Val Gly Val Pro Gly Val Gly Val Pro Gly
    690                     695                     700

Lys Gly Val Pro Gly Val Gly Val Pro Gly Val Gly Val Pro Gly Val
705                     710                     715                     720

Gly Val Pro Gly Ala Gly Ala Gly Ser Gly Ala Gly Ala Gly Ser Gly
                725                     730                     735

Ala Gly Ala Gly Ser Gly Ala Gly Ala Gly Ser Gly Val Gly Val Pro
                740                     745                     750

Gly Val Gly Val Pro Gly Val Gly Val Pro Gly Val Gly Val Pro Gly
                755                     760                     765

Lys Gly Val Pro Gly Val Gly Val Pro Gly Val Gly Val Pro Gly Val
                770                     775                     780

Gly Val Pro Gly Ala Gly Ala Gly Ser Gly Ala Gly Ala Gly Ser Gly
785                     790                     795                     800

Ala Gly Ala Gly Ser Gly Ala Gly Ala Gly Ser Gly Val Gly Val Pro

24

```
                        805                     810                      815


        Gly Val Gly Val Pro Gly Val Gly Val Pro Gly Val Gly Val Pro Gly
                    820                     825                     830


        Lys Gly Val Pro Gly Val Gly Val Pro Gly Val Gly Val Pro Gly Val
                835                     840                     845


        Gly Val Pro Gly Ala Gly Ala Gly Ser Gly Ala Gly Ala Gly Ser Gly
            850                     855                     860


        Ala Gly Ala Met Asp Pro Gly Arg Tyr Gln Asp Leu Arg Ser His His
        865                     870                     875                     880


        His His His His
```

<210> 12
<211> 948
<212> PRT
<213> artificial sequence

<220>
<223> \202r\202d\202k\202o\202O\202j polymer

<400> 12

```
Met Asp Pro Val Val Leu Gln Arg Arg Asp Trp Glu Asn Pro Gly Val
1               5                   10              15

Thr Gln Leu Asn Arg Leu Ala Ala His Pro Pro Phe Ala Ser Asp Pro
            20              25              30

Met Gly Ala Gly Ala Gly Ser Gly Ala Gly Ala Gly Ser Gly Val Gly
        35              40              45

Val Pro Gly Val Gly Val Pro Gly Val Gly Val Pro Gly Val Gly Val
    50              55              60

Pro Gly Lys Gly Val Pro Gly Val Gly Val Pro Gly Val Gly Val Pro
65              70              75              80

Gly Val Gly Val Pro Gly Ala Gly Ala Gly Ser Gly Ala Gly Ala Gly
            85              90              95

Ser Gly Val Gly Val Pro Gly Val Gly Val Pro Gly Val Gly Val Pro
        100             105             110

Gly Val Gly Val Pro Gly Lys Gly Val Pro Gly Val Gly Val Pro Gly
        115             120             125
```

Val Gly Val Pro Gly Val Gly Val Pro Gly Ala Gly Ala Gly Ser Gly
130                 135                 140

Ala Gly Ala Gly Ser Gly Val Gly Val Pro Gly Val Gly Val Pro Gly
145                 150                 155                 160

Val Gly Val Pro Gly Val Gly Val Pro Gly Lys Gly Val Pro Gly Val
165                 170                 175

Gly Val Pro Gly Val Gly Val Pro Gly Val Gly Val Pro Gly Ala Gly
180                 185                 190

Ala Gly Ser Gly Ala Gly Ala Gly Ser Gly Val Gly Val Pro Gly Val
195                 200                 205

Gly Val Pro Gly Val Gly Val Pro Gly Val Gly Val Pro Gly Lys Gly
210                 215                 220

Val Pro Gly Val Gly Val Pro Gly Val Gly Val Pro Gly Val Gly Val
225                 230                 235                 240

Pro Gly Ala Gly Ala Gly Ser Gly Ala Gly Ala Gly Ser Gly Val Gly
245                 250                 255

Val Pro Gly Val Gly Val Pro Gly Val Gly Val Pro Gly Val Gly Val
260                 265                 270

Pro Gly Lys Gly Val Pro Gly Val Gly Val Pro Gly Val Gly Val Pro
275                 280                 285

Gly Val Gly Val Pro Gly Ala Gly Ala Gly Ser Gly Ala Gly Ala Gly
290                 295                 300

Ser Gly Val Gly Val Pro Gly Val Gly Val Pro Gly Val Gly Val Pro
305                 310                 315                 320

Gly Val Gly Val Pro Gly Lys Gly Val Pro Gly Val Gly Val Pro Gly
325                 330                 335

Val Gly Val Pro Gly Val Gly Val Pro Gly Ala Gly Ala Gly Ser Gly
340                 345                 350

Ala Gly Ala Gly Ser Gly Val Gly Val Pro Gly Val Gly Val Pro Gly
355                 360                 365

Val Gly Val Pro Gly Val Gly Val Pro Gly Lys Gly Val Pro Gly Val

```
          370                    375                         380


    Gly Val Pro Gly Val Gly Val Pro Gly Val Gly Val Pro Gly Ala Gly
    385             390             395             400


    Ala Gly Ser Gly Ala Gly Ala Gly Ser Gly Val Gly Val Pro Gly Val
                405             410             415


    Gly Val Pro Gly Val Gly Val Pro Gly Val Gly Val Pro Gly Lys Gly
                420             425             430


    Val Pro Gly Val Gly Val Pro Gly Val Gly Val Pro Gly Val Gly Val
                435             440             445


    Pro Gly Ala Gly Ala Gly Ser Gly Ala Gly Ala Gly Ser Gly Val Gly
                450             455             460


    Val Pro Gly Val Gly Val Pro Gly Val Gly Val Pro Gly Val Gly Val
    465             470             475             480


    Pro Gly Lys Gly Val Pro Gly Val Gly Val Pro Gly Val Gly Val Pro
                485             490             495


    Gly Val Gly Val Pro Gly Ala Gly Ala Gly Ser Gly Ala Gly Ala Gly
                500             505             510


    Ser Gly Val Gly Val Pro Gly Val Gly Val Pro Gly Val Gly Val Pro
                515             520             525


    Gly Val Gly Val Pro Gly Lys Gly Val Pro Gly Val Gly Val Pro Gly
                530             535             540


    Val Gly Val Pro Gly Val Gly Val Pro Gly Ala Gly Ala Gly Ser Gly
    545             550             555             560


    Ala Gly Ala Gly Ser Gly Val Gly Val Pro Gly Val Gly Val Pro Gly
                565             570             575


    Val Gly Val Pro Gly Val Gly Val Pro Gly Lys Gly Val Pro Gly Val
                580             585             590


    Gly Val Pro Gly Val Gly Val Pro Gly Val Gly Val Pro Gly Ala Gly
                595             600             605


    Ala Gly Ser Gly Ala Gly Ala Gly Ser Gly Val Gly Val Pro Gly Val
                610             615             620
```

Gly Val Pro Gly Val Gly Val Pro Gly Val Gly Val Pro Gly Lys Gly
625                     630                 635                     640

Val Pro Gly Val Gly Val Pro Gly Val Gly Val Pro Gly Val Gly Val
                    645                 650                     655

Pro Gly Ala Gly Ala Gly Ser Gly Ala Gly Ala Gly Ser Gly Val Gly
            660                     665                 670

Val Pro Gly Val Gly Val Pro Gly Val Gly Val Pro Gly Val Gly Val
        675                     680                 685

Pro Gly Lys Gly Val Pro Gly Val Gly Val Pro Gly Val Gly Val Pro
    690                     695                 700

Gly Val Gly Val Pro Gly Ala Gly Ala Gly Ser Gly Ala Gly Ala Gly
705                     710                 715                     720

Ser Gly Val Gly Val Pro Gly Val Gly Val Pro Gly Val Gly Val Pro
                    725                 730                     735

Gly Val Gly Val Pro Gly Lys Gly Val Pro Gly Val Gly Val Pro Gly
            740                     745                 750

Val Gly Val Pro Gly Val Gly Val Pro Gly Ala Gly Ala Gly Ser Gly
        755                     760                 765

Ala Gly Ala Gly Ser Gly Val Gly Val Pro Gly Val Gly Val Pro Gly
    770                     775                 780

Val Gly Val Pro Gly Val Gly Val Pro Gly Lys Gly Val Pro Gly Val
785                     790                 795                     800

Gly Val Pro Gly Val Gly Val Pro Gly Val Gly Val Pro Gly Ala Gly
                    805                 810                     815

Ala Gly Ser Gly Ala Gly Ala Gly Ser Gly Val Gly Val Pro Gly Val
            820                     825                 830

Gly Val Pro Gly Val Gly Val Pro Gly Val Gly Val Pro Gly Lys Gly
            835                     840                 845

Val Pro Gly Val Gly Val Pro Gly Val Gly Val Pro Gly Val Gly Val
    850                     855                     860

Pro Gly Ala Gly Ala Gly Ser Gly Ala Gly Ala Gly Ser Gly Val Gly
865                     870                     875                     880

```
Val Pro Gly Val Gly Val Pro Gly Val Gly Val Pro Gly Val Gly Val
            885                 890                 895

Pro Gly Lys Gly Val Pro Gly Val Gly Val Pro Gly Val Gly Val Pro
            900                 905                 910

Gly Val Gly Val Pro Gly Ala Gly Ala Gly Ser Gly Ala Gly Ala Gly
            915                 920                 925

Ser Gly Ala Met Asp Pro Gly Arg Tyr Gln Asp Leu Arg Ser His His
            930                 935                 940

His His His His
945
```

<210> 13
<211> 5
<212> PRT
<213> Artificial Sequence

<220>
<223> amino acid sequence

<400> 13

```
Gly Lys Gly Val Pro
1               5
```

<210> 14
<211> 5
<212> PRT
<213> Artificial Sequence

<220>
<223> amino acid sequence

<400> 14

```
Gly Lys Gly Lys Pro
1               5
```

<210> 15
<211> 5
<212> PRT
<213> Artificial Sequence

<220>
<223> amino acid sequence

<400> 15

```
Gly Lys Gly Arg Pro
1               5
```

<210> 16

<211> 5
<212> PRT
<213> Artificial Sequence

<220>
<223> amino acid sequence

<400> 16

```
Gly Arg Gly Arg Pro
1               5
```

<210> 17
<211> 2690
<212> PRT
<213> Artificial Sequence

<220>
<223> \202d\202k\202o\202P\201D\202P polymer

<400> 17

```
Met Asp Pro Val Val Leu Gln Arg Arg Asp Trp Glu Asn Pro Gly Val
1               5                   10                  15


Thr Gln Leu Asn Arg Leu Ala Ala His Pro Pro Phe Ala Ser Asp Pro
            20                  25                  30


Met Val Pro Gly Val Gly Val Pro Gly Val Gly Val Pro Gly Val Gly
            35                  40                  45


Val Pro Gly Val Gly Gly Ala Gly Ala Gly Ser Val Pro Gly Val Gly
            50                  55                  60


Val Pro Gly Val Gly Val Pro Gly Val Gly Val Pro Gly Val Gly Val
65                  70                  75                  80


Pro Gly Val Gly Val Pro Gly Val Gly Val Pro Gly Val Gly Val Pro
                85                  90                  95


Gly Val Gly Val Pro Gly Val Gly Val Pro Gly Val Gly Val Pro Gly
                100                 105                 110


Val Gly Val Pro Gly Val Gly Gly Ala Gly Ala Gly Ser Val Pro Gly
            115                 120                 125


Val Gly Val Pro Gly Val Gly Val Pro Gly Val Gly Val Pro Gly Val
            130                 135                 140


Gly Val Pro Gly Val Gly Val Pro Gly Val Gly Val Pro Gly Val Gly
145                 150                 155                 160
```

Val Pro Gly Val Gly Val Pro Gly Val Gly Val Pro Gly Val Gly Val
                165                 170                 175

Pro Gly Val Gly Val Pro Gly Val Gly Gly Ala Gly Ala Gly Ser Val
                180                 185                 190

Pro Gly Val Gly Val Pro Gly Val Gly Val Pro Gly Val Gly Val Pro
                195                 200                 205

Gly Val Gly Val Pro Gly Val Gly Val Pro Gly Val Gly Val Pro Gly
    210                 215                 220

Val Gly Val Pro Gly Val Gly Val Pro Gly Val Gly Val Pro Gly Val
225                 230                 235                 240

Gly Val Pro Gly Val Gly Val Pro Gly Val Gly Gly Ala Gly Ala Gly
                245                 250                 255

Ser Val Pro Gly Val Gly Val Pro Gly Val Gly Val Pro Gly Val Gly
                260                 265                 270

Val Pro Gly Val Gly Val Pro Gly Val Gly Val Pro Gly Val Gly Val
    275                 280                 285

Pro Gly Val Gly Val Pro Gly Val Gly Val Pro Gly Val Gly Val Pro
    290                 295                 300

Gly Val Gly Val Pro Gly Val Gly Val Pro Gly Val Gly Gly Ala Gly
305                 310                 315                 320

Ala Gly Ser Val Pro Gly Val Gly Val Pro Gly Val Gly Val Pro Gly
                325                 330                 335

Val Gly Val Pro Gly Val Gly Val Pro Gly Val Gly Val Pro Gly Val
                340                 345                 350

Gly Val Pro Gly Val Gly Val Pro Gly Val Gly Val Pro Gly Val Gly
    355                 360                 365

Val Pro Gly Val Gly Val Pro Gly Val Gly Val Pro Gly Val Gly Gly
    370                 375                 380

Ala Gly Ala Gly Ser Val Pro Gly Val Gly Val Pro Gly Val Gly Val
385                 390                 395                 400

Pro Gly Val Gly Val Pro Gly Val Gly Val Pro Gly Val Gly Val Pro
                405                 410                 415

```
Gly Val Gly Val Pro Gly Val Gly Val Pro Gly Val Gly Val Pro Gly
        420              425              430

Val Gly Val Pro Gly Val Gly Val Pro Gly Val Gly Val Pro Gly Val
        435              440              445

Gly Gly Ala Gly Ala Gly Ser Val Pro Gly Val Gly Val Pro Gly Val
        450              455              460

Gly Val Pro Gly Val Gly Val Pro Gly Val Gly Val Pro Gly Val Gly
465              470              475              480

Val Pro Gly Val Gly Val Pro Gly Val Gly Val Pro Gly Val Gly Val
        485              490              495

Pro Gly Val Gly Val Pro Gly Val Gly Val Pro Gly Val Gly Val Pro
        500              505              510

Gly Val Gly Gly Ala Gly Ala Gly Ser Val Pro Gly Val Gly Val Pro
        515              520              525

Gly Val Gly Val Pro Gly Val Gly Val Pro Gly Val Gly Val Pro Gly
        530              535              540

Val Gly Val Pro Gly Val Gly Val Pro Gly Val Gly Val Pro Gly Val
545              550              555              560

Gly Val Pro Gly Val Gly Val Pro Gly Val Gly Val Pro Gly Val Gly
                565              570              575

Val Pro Gly Val Gly Gly Ala Gly Ala Gly Ser Val Pro Gly Val Gly
        580              585              590

Val Pro Gly Val Gly Val Pro Gly Val Gly Val Pro Gly Val Gly Val
        595              600              605

Pro Gly Val Gly Val Pro Gly Val Gly Val Pro Gly Val Gly Val Pro
610              615              620

Gly Val Gly Val Pro Gly Val Gly Val Pro Gly Val Gly Val Pro Gly
625              630              635              640

Val Gly Val Pro Gly Val Gly Gly Ala Gly Ala Gly Ser Val Pro Gly
        645              650              655

Val Gly Val Pro Gly Val Gly Val Pro Gly Val Gly Val Pro Gly Val
```

<pre>
                        660                    665                        670


        Gly Val Pro Gly Val Gly Val Pro Gly Val Gly Val Pro Gly Val Gly
                    675              680                   685


        Val Pro Gly Val Gly Val Pro Gly Val Gly Val Pro Gly Val Gly Val
            690              695                   700


        Pro Gly Val Gly Val Pro Gly Val Gly Gly Ala Gly Ala Gly Ser Val
        705                  710              715                   720


        Pro Gly Val Gly Val Pro Gly Val Gly Val Pro Gly Val Gly Val Pro
                    725              730                   735


        Gly Val Gly Val Pro Gly Val Gly Val Pro Gly Val Gly Val Pro Gly
                    740              745                   750


        Val Gly Val Pro Gly Val Gly Val Pro Gly Val Gly Val Pro Gly Val
            755              760                   765


        Gly Val Pro Gly Val Gly Val Pro Gly Val Gly Gly Ala Gly Ala Gly
            770              775                   780


        Ser Val Pro Gly Val Gly Val Pro Gly Val Gly Val Pro Gly Val Gly
        785                  790                   795                   800


        Val Pro Gly Val Gly Val Pro Gly Val Gly Val Pro Gly Val Gly Val
                    805              810                   815


        Pro Gly Val Gly Val Pro Gly Val Gly Val Pro Gly Val Gly Val Pro
                    820              825                   830


        Gly Val Gly Val Pro Gly Val Gly Val Pro Gly Val Gly Gly Ala Gly
                    835              840                   845


        Ala Gly Ser Val Pro Gly Val Gly Val Pro Gly Val Gly Val Pro Gly
            850              855                   860


        Val Gly Val Pro Gly Val Gly Val Pro Gly Val Gly Val Pro Gly Val
        865              870                   875                   880


        Gly Val Pro Gly Val Gly Val Pro Gly Val Gly Val Pro Gly Val Gly
                    885                   890                   895


        Val Pro Gly Val Gly Val Pro Gly Val Gly Val Pro Gly Val Gly Gly
                    900                   905                   910
</pre>

```
Ala Gly Ala Gly Ser Val Pro Gly Val Gly Val Pro Gly Val Gly Val
        915                 920                 925

Pro Gly Val Gly Val Pro Gly Val Gly Val Pro Gly Val Gly Val Pro
    930                 935                 940

Gly Val Gly Val Pro Gly Val Gly Val Pro Gly Val Gly Val Pro Gly
945                 950                 955                 960

Val Gly Val Pro Gly Val Gly Val Pro Gly Val Gly Val Pro Gly Val
            965                 970                 975

Gly Gly Ala Gly Ala Gly Ser Val Pro Gly Val Gly Val Pro Gly Val
        980                 985                 990

Gly Val Pro Gly Val Gly Val Pro  Gly Val Gly Val Pro  Gly Val Gly
        995             1000                1005

Val Pro  Gly Val Gly Val Pro  Gly Val Gly Val Pro  Gly Val Gly
    1010            1015                1020

Val Pro  Gly Val Gly Val Pro  Gly Val Gly Val Pro  Gly Val Gly
    1025            1030                1035

Val Pro  Gly Val Gly Gly Ala  Gly Ala Gly Ser Val  Pro Gly Val
    1040            1045                1050

Gly Val  Pro Gly Val Gly Val  Pro Gly Val Gly Val  Pro Gly Val
    1055            1060                1065

Gly Val  Pro Gly Val Gly Val  Pro Gly Val Gly Val  Pro Gly Val
    1070            1075                1080

Gly Val  Pro Gly Val Gly Val  Pro Gly Val Gly Val  Pro Gly Val
    1085            1090                1095

Gly Val  Pro Gly Val Gly Val  Pro Gly Val Gly Gly  Ala Gly Ala
    1100            1105                1110

Gly Ser  Val Pro Gly Val Gly  Val Pro Gly Val Gly  Val Pro Gly
    1115            1120                1125

Val Gly  Val Pro Gly Val Gly  Val Pro Gly Val Gly  Val Pro Gly
    1130            1135                1140

Val Gly  Val Pro Gly Val Gly  Val Pro Gly Val Gly  Val Pro Gly
    1145            1150                1155
```

35

Val Gly Val Pro Gly Val Gly Val Pro Gly Val Gly Val Pro Gly
1160 1165 1170

Val Gly Gly Ala Gly Ala Gly Ser Val Pro Gly Val Gly Val Pro
1175 1180 1185

Gly Val Gly Val Pro Gly Val Gly Val Pro Gly Val Gly Val Pro
1190 1195 1200

Gly Val Gly Val Pro Gly Val Gly Val Pro Gly Val Gly Val Pro
1205 1210 1215

Gly Val Gly Val Pro Gly Val Gly Val Pro Gly Val Gly Val Pro
1220 1225 1230

Gly Val Gly Val Pro Gly Val Gly Gly Ala Gly Ala Gly Ser Val
1235 1240 1245

Pro Gly Val Gly Val Pro Gly Val Gly Val Pro Gly Val Gly Val
1250 1255 1260

Pro Gly Val Gly Val Pro Gly Val Gly Val Pro Gly Val Gly Val
1265 1270 1275

Pro Gly Val Gly Val Pro Gly Val Gly Val Pro Gly Val Gly Val
1280 1285 1290

Pro Gly Val Gly Val Pro Gly Val Gly Val Pro Gly Val Gly Gly
1295 1300 1305

Ala Gly Ala Gly Ser Val Pro Gly Val Gly Val Pro Gly Val Gly
1310 1315 1320

Val Pro Gly Val Gly Val Pro Gly Val Gly Val Pro Gly Val Gly
1325 1330 1335

Val Pro Gly Val Gly Val Pro Gly Val Gly Val Pro Gly Val Gly
1340 1345 1350

Val Pro Gly Val Gly Val Pro Gly Val Gly Val Pro Gly Val Gly
1355 1360 1365

Val Pro Gly Val Gly Gly Ala Gly Ala Gly Ser Val Pro Gly Val
1370 1375 1380

Gly Val Pro Gly Val Gly Val Pro Gly Val Gly Val Pro Gly Val
1385 1390 1395

```
Gly Val  Pro Gly Val Gly Val  Pro Gly Val Gly Val  Pro Gly Val
    1400             1405             1410

Gly Val  Pro Gly Val Gly Val  Pro Gly Val Gly Val  Pro Gly Val
    1415             1420             1425

Gly Val  Pro Gly Val Gly Val  Pro Gly Val Gly Gly  Ala Gly Ala
    1430             1435             1440

Gly Ser  Val Pro Gly Val Gly  Val Pro Gly Val Gly  Val Pro Gly
    1445             1450             1455

Val Gly  Val Pro Gly Val Gly  Val Pro Gly Val Gly  Val Pro Gly
    1460             1465             1470

Val Gly  Val Pro Gly Val Gly  Val Pro Gly Val Gly  Val Pro Gly
    1475             1480             1485

Val Gly  Val Pro Gly Val Gly  Val Pro Gly Val Gly  Val Pro Gly
    1490             1495             1500

Val Gly  Gly Ala Gly Ala Gly  Ser Val Pro Gly Val  Gly Val Pro
    1505             1510             1515

Gly Val  Gly Val Pro Gly Val  Gly Val Pro Gly Val  Gly Val Pro
    1520             1525             1530

Gly Val  Gly Val Pro Gly Val  Gly Val Pro Gly Val  Gly Val Pro
    1535             1540             1545

Gly Val  Gly Val Pro Gly Val  Gly Val Pro Gly Val  Gly Val Pro
    1550             1555             1560

Gly Val  Gly Val Pro Gly Val  Gly Gly Ala Gly Ala  Gly Ser Val
    1565             1570             1575

Pro Gly  Val Gly Val Pro Gly  Val Gly Val Pro Gly  Val Gly Val
    1580             1585             1590

Pro Gly  Val Gly Val Pro Gly  Val Gly Val Pro Gly  Val Gly Val
    1595             1600             1605

Pro Gly  Val Gly Val Pro Gly  Val Gly Val Pro Gly  Val Gly Val
    1610             1615             1620

Pro Gly  Val Gly Val Pro Gly  Val Gly Val Pro Gly  Val Gly Gly
```

1625        1630        1635

Ala Gly Ala Gly Ser Val Pro Gly Val Gly Val Pro Gly Val Gly
1640        1645        1650

Val Pro Gly Val Gly Val Pro Gly Val Gly Val Pro Gly Val Gly
1655        1660        1665

Val Pro Gly Val Gly Val Pro Gly Val Gly Val Pro Gly Val Gly
1670        1675        1680

Val Pro Gly Val Gly Val Pro Gly Val Gly Val Pro Gly Val Gly
1685        1690        1695

Val Pro Gly Val Gly Gly Ala Gly Ala Gly Ser Val Pro Gly Val
1700        1705        1710

Gly Val Pro Gly Val Gly Val Pro Gly Val Gly Val Pro Gly Val
1715        1720        1725

Gly Val Pro Gly Val Gly Val Pro Gly Val Gly Val Pro Gly Val
1730        1735        1740

Gly Val Pro Gly Val Gly Val Pro Gly Val Gly Val Pro Gly Val
1745        1750        1755

Gly Val Pro Gly Val Gly Val Pro Gly Val Gly Gly Ala Gly Ala
1760        1765        1770

Gly Ser Val Pro Gly Val Gly Val Pro Gly Val Gly Val Pro Gly
1775        1780        1785

Val Gly Val Pro Gly Val Gly Val Pro Gly Val Gly Val Pro Gly
1790        1795        1800

Val Gly Val Pro Gly Val Gly Val Pro Gly Val Gly Val Pro Gly
1805        1810        1815

Val Gly Val Pro Gly Val Gly Val Pro Gly Val Gly Val Pro Gly
1820        1825        1830

Val Gly Gly Ala Gly Ala Gly Ser Val Pro Gly Val Gly Val Pro
1835        1840        1845

Gly Val Gly Val Pro Gly Val Gly Val Pro Gly Val Gly Val Pro
1850        1855        1860

```
Gly Val  Gly Val Pro Gly Val  Gly Val Pro Gly Val  Gly Val Pro
    1865             1870             1875

Gly Val  Gly Val Pro Gly Val  Gly Val Pro Gly Val  Gly Val Pro
    1880             1885             1890

Gly Val  Gly Val Pro Gly Val  Gly Gly Ala Gly Ala  Gly Ser Val
    1895             1900             1905

Pro Gly  Val Gly Val Pro Gly  Val Gly Val Pro Gly  Val Gly Val
    1910             1915             1920

Pro Gly  Val Gly Val Pro Gly  Val Gly Val Pro Gly  Val Gly Val
    1925             1930             1935

Pro Gly  Val Gly Val Pro Gly  Val Gly Val Pro Gly  Val Gly Val
    1940             1945             1950

Pro Gly  Val Gly Val Pro Gly  Val Gly Val Pro Gly  Val Gly Gly
    1955             1960             1965

Ala Gly  Ala Gly Ser Val Pro  Gly Val Gly Val Pro  Gly Val Gly
    1970             1975             1980

Val Pro  Gly Val Gly Val Pro  Gly Val Gly Val Pro  Gly Val Gly
    1985             1990             1995

Val Pro  Gly Val Gly Val Pro  Gly Val Gly Val Pro  Gly Val Gly
    2000             2005             2010

Val Pro  Gly Val Gly Val Pro  Gly Val Gly Val Pro  Gly Val Gly
    2015             2020             2025

Val Pro  Gly Val Gly Gly Ala  Gly Ala Gly Ser Val  Pro Gly Val
    2030             2035             2040

Gly Val  Pro Gly Val Gly Val  Pro Gly Val Gly Val  Pro Gly Val
    2045             2050             2055

Gly Val  Pro Gly Val Gly Val  Pro Gly Val Gly Val  Pro Gly Val
    2060             2065             2070

Gly Val  Pro Gly Val Gly Val  Pro Gly Val Gly Val  Pro Gly Val
    2075             2080             2085

Gly Val  Pro Gly Val Gly Val  Pro Gly Val Gly Gly  Ala Gly Ala
    2090             2095             2100
```

```
Gly Ser Val Pro Gly Val Gly  Val Pro Gly Val Gly  Val Pro Gly
    2105              2110              2115

Val Gly Val Pro Gly Val Gly  Val Pro Gly Val Gly  Val Pro Gly
    2120              2125              2130

Val Gly Val Pro Gly Val Gly  Val Pro Gly Val Gly  Val Pro Gly
    2135              2140              2145

Val Gly Val Pro Gly Val Gly  Val Pro Gly Val Gly  Val Pro Gly
    2150              2155              2160

Val Gly Gly Ala Gly Ala Gly  Ser Val Pro Gly Val  Gly Val Pro
    2165              2170              2175

Gly Val Gly Val Pro Gly Val  Gly Val Pro Gly Val  Gly Val Pro
    2180              2185              2190

Gly Val Gly Val Pro Gly Val  Gly Val Pro Gly Val  Gly Val Pro
    2195              2200              2205

Gly Val Gly Val Pro Gly Val  Gly Val Pro Gly Val  Gly Val Pro
    2210              2215              2220

Gly Val Gly Val Pro Gly Val  Gly Gly Ala Gly Ala  Gly Ser Val
    2225              2230              2235

Pro Gly Val Gly Val Pro Gly  Val Gly Val Pro Gly  Val Gly Val
    2240              2245              2250

Pro Gly Val Gly Val Pro Gly  Val Gly Val Pro Gly  Val Gly Val
    2255              2260              2265

Pro Gly Val Gly Val Pro Gly  Val Gly Val Pro Gly  Val Gly Val
    2270              2275              2280

Pro Gly Val Gly Val Pro Gly  Val Gly Val Pro Gly  Val Gly Gly
    2285              2290              2295

Ala Gly Ala Gly Ser Val Pro  Gly Val Gly Val Pro  Gly Val Gly
    2300              2305              2310

Val Pro Gly Val Gly Val Pro  Gly Val Gly Val Pro  Gly Val Gly
    2315              2320              2325

Val Pro Gly Val Gly Val Pro  Gly Val Gly Val Pro  Gly Val Gly
    2330              2335              2340
```

```
Val Pro  Gly Val Gly Val Pro  Gly Val Gly Val Pro  Gly Val Gly
    2345                 2350                 2355

Val Pro  Gly Val Gly Gly Ala  Gly Ala Gly Ser Val  Pro Gly Val
    2360                 2365                 2370

Gly Val  Pro Gly Val Gly Val  Pro Gly Val Gly Val  Pro Gly Val
    2375                 2380                 2385

Gly Val  Pro Gly Val Gly Val  Pro Gly Val Gly Val  Pro Gly Val
    2390                 2395                 2400

Gly Val  Pro Gly Val Gly Val  Pro Gly Val Gly Val  Pro Gly Val
    2405                 2410                 2415

Gly Val  Pro Gly Val Gly Val  Pro Gly Val Gly Gly  Ala Gly Ala
    2420                 2425                 2430

Gly Ser  Val Pro Gly Val Gly  Val Pro Gly Val Gly  Val Pro Gly
    2435                 2440                 2445

Val Gly  Val Pro Gly Val Gly  Val Pro Gly Val Gly  Val Pro Gly
    2450                 2455                 2460

Val Gly  Val Pro Gly Val Gly  Val Pro Gly Val Gly  Val Pro Gly
    2465                 2470                 2475

Val Gly  Val Pro Gly Val Gly  Val Pro Gly Val Gly  Val Pro Gly
    2480                 2485                 2490

Val Gly  Gly Ala Gly Ala Gly  Ser Val Pro Gly Val  Gly Val Pro
    2495                 2500                 2505

Gly Val  Gly Val Pro Gly Val  Gly Val Pro Gly Val  Gly Val Pro
    2510                 2515                 2520

Gly Val  Gly Val Pro Gly Val  Gly Val Pro Gly Val  Gly Val Pro
    2525                 2530                 2535

Gly Val  Gly Val Pro Gly Val  Gly Val Pro Gly Val  Gly Val Pro
    2540                 2545                 2550

Gly Val  Gly Val Pro Gly Val  Gly Gly Ala Gly Ala  Gly Ser Val
    2555                 2560                 2565

Pro Gly  Val Gly Val Pro Gly  Val Gly Val Pro Gly  Val Gly Val
```

                2570                    2575                      2580


      Pro Gly  Val Gly Val Pro Gly  Val Gly Val Pro Gly  Val Gly Val
          2585                    2590                      2595


      Pro Gly  Val Gly Val Pro Gly  Val Gly Val Pro Gly  Val Gly Val
          2600                    2605                      2610


      Pro Gly  Val Gly Val Pro Gly  Val Gly Val Pro Gly  Val Gly Gly
          2615                    2620                      2625


      Ala Gly  Ala Gly Ser Val Pro  Gly Val Gly Val Pro  Gly Val Gly
          2630                    2635                      2640


      Val Pro  Gly Val Gly Val Pro  Gly Val Gly Val Pro  Gly Val Gly
          2645                    2650                      2655


      Val Pro  Gly Val Gly Val Pro  Gly Val Gly Val Pro  Gly Val Gly
          2660                    2665                      2670


      Met Asp  Pro Gly Arg Tyr Gln  Asp Leu Arg Ser His  His His His
          2675                    2680                      2685


      His His
          2690

<210> 18
<211> 1384
<212> PRT
<213> Artificial Sequence

<220>
<223> \202r\202k\202o\202S\201D\202P polymer

<400> 18

```
Met Asp Pro Val Val Leu Gln Arg Arg Asp Trp Glu Asn Pro Gly Val
1               5               10                  15

Thr Gln Leu Asn Arg Leu Ala Ala His Pro Pro Phe Ala Ser Asp Pro
        20              25                  30

Met Gly Ala Gly Ala Gly Ser Gly Ala Gly Ala Gly Ser Gly Ala Gly
        35              40                  45

Ala Gly Ser Gly Ala Gly Ala Gly Ser Gly Ala Gly Ala Gly Ser Gly
    50              55                  60

Ala Gly Ala Gly Ser Gly Val Gly Val Pro Gly Val Gly Val Pro Gly
65              70                  75                  80
```

```
Ala Gly Ala Gly Ser Gly Ala Gly Ala Gly Ser Gly Ala Gly Ala Gly
                85                    90                    95

Ser Gly Ala Gly Ala Gly Ser Gly Ala Gly Ala Gly Ser Gly Ala Gly
               100                   105                   110

Ala Gly Ser Gly Val Gly Val Pro Gly Val Gly Val Pro Gly Ala Gly
               115                   120                   125

Ala Gly Ser Gly Ala Gly Ala Gly Ser Gly Ala Gly Ala Gly Ser Gly
               130                   135                   140

Ala Gly Ala Gly Ser Gly Ala Gly Ala Gly Ser Gly Ala Gly Ala Gly
145                   150                   155                   160

Ser Gly Val Gly Val Pro Gly Val Gly Val Pro Gly Ala Gly Ala Gly
               165                   170                   175

Ser Gly Ala Gly Ala Gly Ser Gly Ala Gly Ala Gly Ser Gly Ala Gly
               180                   185                   190

Ala Gly Ser Gly Ala Gly Ala Gly Ser Gly Ala Gly Ala Gly Ser Gly
               195                   200                   205

Val Gly Val Pro Gly Val Gly Val Pro Gly Ala Gly Ala Gly Ser Gly
               210                   215                   220

Ala Gly Ala Gly Ser Gly Ala Gly Ala Gly Ser Gly Ala Gly Ala Gly
225                   230                   235                   240

Ser Gly Ala Gly Ala Gly Ser Gly Ala Gly Ala Gly Ser Gly Val Gly
               245                   250                   255

Val Pro Gly Val Gly Val Pro Gly Ala Gly Ala Gly Ser Gly Ala Gly
               260                   265                   270

Ala Gly Ser Gly Ala Gly Ala Gly Ser Gly Ala Gly Ala Gly Ser Gly
               275                   280                   285

Ala Gly Ala Gly Ser Gly Ala Gly Ala Gly Ser Gly Val Gly Val Pro
               290                   295                   300

Gly Val Gly Val Pro Gly Ala Gly Ala Gly Ser Gly Ala Gly Ala Gly
305                   310                   315                   320

Ser Gly Ala Gly Ala Gly Ser Gly Ala Gly Ala Gly Ser Gly Ala Gly
```

44

                    325                         330                         335

    Ala Gly Ser Gly Ala Gly Ala Gly Ser Gly Val Gly Val Pro Gly Val
                340                         345                         350

    Gly Val Pro Gly Ala Gly Ala Gly Ser Gly Ala Gly Ala Gly Ser Gly
                355                         360                         365

    Ala Gly Ala Gly Ser Gly Ala Gly Ala Gly Ser Gly Ala Gly Ala Gly
            370                         375                         380

    Ser Gly Ala Gly Ala Gly Ser Gly Val Gly Val Pro Gly Val Gly Val
    385                         390                         395                         400

    Pro Gly Ala Gly Ala Gly Ser Gly Ala Gly Ala Gly Ser Gly Ala Gly
                            405                         410                         415

    Ala Gly Ser Gly Ala Gly Ala Gly Ser Gly Ala Gly Ala Gly Ser Gly
                420                         425                         430

    Ala Gly Ala Gly Ser Gly Val Gly Val Pro Gly Val Gly Val Pro Gly
                435                         440                         445

    Ala Gly Ala Gly Ser Gly Ala Gly Ala Gly Ser Gly Ala Gly Ala Gly
            450                         455                         460

    Ser Gly Ala Gly Ala Gly Ser Gly Ala Gly Ala Gly Ser Gly Ala Gly
    465                         470                         475                         480

    Ala Gly Ser Gly Val Gly Val Pro Gly Val Gly Val Pro Gly Ala Gly
                485                         490                         495

    Ala Gly Ser Gly Ala Gly Ala Gly Ser Gly Ala Gly Ala Gly Ser Gly
                500                         505                         510

    Ala Gly Ala Gly Ser Gly Ala Gly Ala Gly Ser Gly Ala Gly Ala Gly
            515                         520                         525

    Ser Gly Val Gly Val Pro Gly Val Gly Val Pro Gly Ala Gly Ala Gly
            530                         535                         540

    Ser Gly Ala Gly Ala Gly Ser Gly Ala Gly Ala Gly Ser Gly Ala Gly
    545                         550                         555                         560

    Ala Gly Ser Gly Ala Gly Ala Gly Ser Gly Ala Gly Ala Gly Ser Gly
                565                         570                         575

Val Gly Val Pro Gly Val Gly Val Pro Gly Ala Gly Ala Gly Ser Gly
        580             585             590

Ala Gly Ala Gly Ser Gly Ala Gly Ala Gly Ser Gly Ala Gly Ala Gly
        595             600             605

Ser Gly Ala Gly Ala Gly Ser Gly Ala Gly Ala Gly Ser Gly Val Gly
        610             615             620

Val Pro Gly Val Gly Val Pro Gly Ala Gly Ala Gly Ser Gly Ala Gly
625             630             635             640

Ala Gly Ser Gly Ala Gly Ala Gly Ser Gly Ala Gly Ala Gly Ser Gly
        645             650             655

Ala Gly Ala Gly Ser Gly Ala Gly Ala Gly Ser Gly Val Gly Val Pro
        660             665             670

Gly Val Gly Val Pro Gly Ala Gly Ala Gly Ser Gly Ala Gly Ala Gly
        675             680             685

Ser Gly Ala Gly Ala Gly Ser Gly Ala Gly Ala Gly Ser Gly Ala Gly
        690             695             700

Ala Gly Ser Gly Ala Gly Ala Gly Ser Gly Val Gly Val Pro Gly Val
705             710             715             720

Gly Val Pro Gly Ala Gly Ala Gly Ser Gly Ala Gly Ala Gly Ser Gly
        725             730             735

Ala Gly Ala Gly Ser Gly Ala Gly Ala Gly Ser Gly Ala Gly Ala Gly
        740             745             750

Ser Gly Ala Gly Ala Gly Ser Gly Val Gly Val Pro Gly Val Gly Val
        755             760             765

Pro Gly Ala Gly Ala Gly Ser Gly Ala Gly Ala Gly Ser Gly Ala Gly
        770             775             780

Ala Gly Ser Gly Ala Gly Ala Gly Ser Gly Ala Gly Ala Gly Ser Gly
785             790             795             800

Ala Gly Ala Gly Ser Gly Val Gly Val Pro Gly Val Gly Val Pro Gly
        805             810             815

Ala Gly Ala Gly Ser Gly Ala Gly Ala Gly Ser Gly Ala Gly Ala Gly
        820             825             830

Ser Gly Ala Gly Ala Gly Ser Gly Ala Gly Ala Gly Ser Gly Ala Gly
        835                 840                 845

Ala Gly Ser Gly Val Gly Val Pro Gly Val Gly Val Pro Gly Ala Gly
        850                 855                 860

Ala Gly Ser Gly Ala Gly Ala Gly Ser Gly Ala Gly Ala Gly Ser Gly
865                 870                 875                 880

Ala Gly Ala Gly Ser Gly Ala Gly Ala Gly Ser Gly Ala Gly Ala Gly
            885                 890                 895

Ser Gly Val Gly Val Pro Gly Val Gly Val Pro Gly Ala Gly Ala Gly
            900                 905                 910

Ser Gly Ala Gly Ala Gly Ser Gly Ala Gly Ala Gly Ser Gly Ala Gly
        915                 920                 925

Ala Gly Ser Gly Ala Gly Ala Gly Ser Gly Ala Gly Ala Gly Ser Gly
    930                 935                 940

Val Gly Val Pro Gly Val Gly Val Pro Gly Ala Gly Ala Gly Ser Gly
945                 950                 955                 960

Ala Gly Ala Gly Ser Gly Ala Gly Ala Gly Ser Gly Ala Gly Ala Gly
            965                 970                 975

Ser Gly Ala Gly Ala Gly Ser Gly Ala Gly Ala Gly Ser Gly Val Gly
            980                 985                 990

Val Pro Gly Val Gly Val Pro Gly Ala Gly Ala Gly Ser  Gly Ala Gly
        995                 1000                1005

Ala Gly  Ser Gly Ala Gly Ala  Gly Ser Gly Ala Gly  Ala Gly Ser
    1010                1015                1020

Gly Ala  Gly Ala Gly Ser Gly  Ala Gly Ala Gly Ser  Gly Val Gly
    1025                1030                1035

Val Pro  Gly Val Gly Val Pro  Gly Ala Gly Ala Gly  Ser Gly Ala
    1040                1045                1050

Gly Ala  Gly Ser Gly Ala Gly  Ala Gly Ser Gly Ala  Gly Ala Gly
    1055                1060                1065

Ser Gly  Ala Gly Ala Gly Ser  Gly Ala Gly Ala Gly  Ser Gly Val
    1070                1075                1080

```
Gly Val  Pro Gly Val Gly Val  Pro Gly Ala Gly Ala  Gly Ser Gly
    1085                 1090                 1095

Ala Gly  Ala Gly Ser Gly Ala  Gly Ala Gly Ser Gly  Ala Gly Ala
    1100                 1105                 1110

Gly Ser  Gly Ala Gly Ala Gly  Ser Gly Ala Gly Ala  Gly Ser Gly
    1115                 1120                 1125

Val Gly  Val Pro Gly Val Gly  Val Pro Gly Ala Gly  Ala Gly Ser
    1130                 1135                 1140

Gly Ala  Gly Ala Gly Ser Gly  Ala Gly Ala Gly Ser  Gly Ala Gly
    1145                 1150                 1155

Ala Gly  Ser Gly Ala Gly Ala  Gly Ser Gly Ala Gly  Ala Gly Ser
    1160                 1165                 1170

Gly Val  Gly Val Pro Gly Val  Gly Val Pro Gly Ala  Gly Ala Gly
    1175                 1180                 1185

Ser Gly  Ala Gly Ala Gly Ser  Gly Ala Gly Ala Gly  Ser Gly Ala
    1190                 1195                 1200

Gly Ala  Gly Ser Gly Ala Gly  Ala Gly Ser Gly Ala  Gly Ala Gly
    1205                 1210                 1215

Ser Gly  Val Gly Val Pro Gly  Val Gly Val Pro Gly  Ala Gly Ala
    1220                 1225                 1230

Gly Ser  Gly Ala Gly Ala Gly  Ser Gly Ala Gly Ala  Gly Ser Gly
    1235                 1240                 1245

Ala Gly  Ala Gly Ser Gly Ala  Gly Ala Gly Ser Gly  Ala Gly Ala
    1250                 1255                 1260

Gly Ser  Gly Val Gly Val Pro  Gly Val Gly Val Pro  Gly Ala Gly
    1265                 1270                 1275

Ala Gly  Ser Gly Ala Gly Ala  Gly Ser Gly Ala Gly  Ala Gly Ser
    1280                 1285                 1290

Gly Ala  Gly Ala Gly Ser Gly  Ala Gly Ala Gly Ser  Gly Ala Gly
    1295                 1300                 1305

Ala Gly  Ser Gly Val Gly Val  Pro Gly Val Gly Val  Pro Gly Ala
```

```
            1310                    1315                    1320


        Gly Ala  Gly Ser Gly Ala Gly  Ala Gly Ser Gly Ala  Gly Ala Gly
            1325                    1330                    1335


        Ser Gly  Ala Gly Ala Gly Ser  Gly Ala Gly Ala Gly  Ser Gly Ala
            1340                    1345                    1350


        Gly Ala  Gly Ser Gly Val Gly  Val Pro Gly Val Gly  Val Pro Met
            1355                    1360                    1365


        Asp Pro  Gly Arg Tyr Gln Asp  Leu Arg Ser His His  His His His
            1370                    1375                    1380


        His
```

<210> 19
<211> 1094
<212> PRT
<213> Artificial Sequence

<220>
<223> \202r\202k\202o\202S polymer

<400> 19

```
Met Asp Pro Val Val Leu Gln Arg Arg Asp Trp Glu Asn Pro Gly Val
1               5                   10                  15

Thr Gln Leu Asn Arg Leu Ala Ala His Pro Pro Phe Ala Ser Asp Pro
        20                  25                  30

Met Gly Ala Gly Ala Gly Ser Gly Ala Gly Ala Gly Ser Gly Ala Gly
        35                  40                  45

Ala Gly Ser Gly Ala Gly Ala Gly Ser Gly Ala Gly Ala Gly Ser Gly
    50                  55                  60

Ala Gly Ala Gly Ser Gly Ala Gly Ala Gly Ser Gly Ala Gly Ala Gly
65                  70                  75                  80

Ser Gly Ala Gly Ala Gly Ser Gly Ala Gly Ala Gly Ser Gly Ala Gly
            85                  90                  95

Ala Gly Ser Gly Ala Gly Ala Gly Ser Gly Ala Gly Ala Gly Ser Gly
        100                 105                 110

Ala Gly Ala Gly Ser Gly Ala Gly Ala Gly Ser Gly Ala Gly Ala Gly
        115                 120                 125
```

Ser Gly Ala Gly Ala Gly Ser Gly Ala Gly Ala Gly Ser Gly Ala Gly
    130             135             140

Ala Gly Ser Gly Ala Gly Ala Gly Ser Gly Ala Gly Ala Gly Ser Gly
    145             150             155             160

Ala Gly Ala Gly Ser Gly Ala Gly Ala Gly Ser Gly Ala Gly Ala Gly
        165             170             175

Ser Gly Ala Gly Ala Gly Ser Gly Ala Gly Ala Gly Ser Gly Ala Gly
        180             185             190

Ala Gly Ser Gly Ala Gly Ala Gly Ser Gly Ala Gly Ala Gly Ser Gly
        195             200             205

Ala Gly Ala Gly Ser Gly Ala Gly Ala Gly Ser Gly Ala Gly Ala Gly
    210             215             220

Ser Gly Ala Gly Ala Gly Ser Gly Ala Gly Ala Gly Ser Gly Ala Gly
    225             230             235             240

Ala Gly Ser Gly Ala Gly Ala Gly Ser Gly Ala Gly Ala Gly Ser Gly
        245             250             255

Ala Gly Ala Gly Ser Gly Ala Gly Ala Gly Ser Gly Ala Gly Ala Gly
        260             265             270

Ser Gly Ala Gly Ala Gly Ser Gly Ala Gly Ala Gly Ser Gly Ala Gly
        275             280             285

Ala Gly Ser Gly Ala Gly Ala Gly Ser Gly Ala Gly Ala Gly Ser Gly
        290             295             300

Ala Gly Ala Gly Ser Gly Ala Gly Ala Gly Ser Gly Ala Gly Ala Gly
    305             310             315             320

Ser Gly Ala Gly Ala Gly Ser Gly Ala Gly Ala Gly Ser Gly Ala Gly
        325             330             335

Ala Gly Ser Gly Ala Gly Ala Gly Ser Gly Ala Gly Ala Gly Ser Gly
        340             345             350

Ala Gly Ala Gly Ser Gly Ala Gly Ala Gly Ser Gly Ala Gly Ala Gly
    355             360             365

Ser Gly Ala Gly Ala Gly Ser Gly Ala Gly Ala Gly Ser Gly Ala Gly

<pre>
           370                      375                      380


        Ala Gly Ser Gly Ala Gly Ala Gly Ser Gly Ala Gly Ala Gly Ser Gly
        385                 390                 395                 400


        Ala Gly Ala Gly Ser Gly Ala Gly Ala Gly Ser Gly Ala Gly Ala Gly
                    405                 410                 415


        Ser Gly Ala Gly Ala Gly Ser Gly Ala Gly Ala Gly Ser Gly Ala Gly
                    420                 425                 430


        Ala Gly Ser Gly Ala Gly Ala Gly Ser Gly Ala Gly Ala Gly Ser Gly
                    435                 440                 445


        Ala Gly Ala Gly Ser Gly Ala Gly Ala Gly Ser Gly Ala Gly Ala Gly
                    450                 455                 460


        Ser Gly Ala Gly Ala Gly Ser Gly Ala Gly Ala Gly Ser Gly Ala Gly
        465                 470                 475                 480


        Ala Gly Ser Gly Ala Gly Ala Gly Ser Gly Ala Gly Ala Gly Ser Gly
                    485                 490                 495


        Ala Gly Ala Gly Ser Gly Ala Gly Ala Gly Ser Gly Ala Gly Ala Gly
                    500                 505                 510


        Ser Gly Ala Gly Ala Gly Ser Gly Ala Gly Ala Gly Ser Gly Ala Gly
                    515                 520                 525


        Ala Gly Ser Gly Ala Gly Ala Gly Ser Gly Ala Gly Ala Gly Ser Gly
                    530                 535                 540


        Ala Gly Ala Gly Ser Gly Ala Gly Ala Gly Ser Gly Ala Gly Ala Gly
        545                 550                 555                 560


        Ser Gly Ala Gly Ala Gly Ser Gly Ala Gly Ala Gly Ser Gly Ala Gly
                    565                 570                 575


        Ala Gly Ser Gly Ala Gly Ala Gly Ser Gly Ala Gly Ala Gly Ser Gly
                    580                 585                 590


        Ala Gly Ala Gly Ser Gly Ala Gly Ala Gly Ser Gly Ala Gly Ala Gly
                    595                 600                 605


        Ser Gly Ala Gly Ala Gly Ser Gly Ala Gly Ala Gly Ser Gly Ala Gly
                    610                 615                 620
</pre>

Ala Gly Ser Gly Ala Gly Ala Gly Ser Gly Ala Gly Ala Gly Ser Gly
625                 630             635                 640

Ala Gly Ala Gly Ser Gly Ala Gly Ala Gly Ser Gly Ala Gly Ala Gly
                645             650                 655

Ser Gly Ala Gly Ala Gly Ser Gly Ala Gly Ala Gly Ser Gly Ala Gly
        660             665                 670

Ala Gly Ser Gly Ala Gly Ala Gly Ser Gly Ala Gly Ala Gly Ser Gly
        675             680                 685

Ala Gly Ala Gly Ser Gly Ala Gly Ala Gly Ser Gly Ala Gly Ala Gly
        690             695                 700

Ser Gly Ala Gly Ala Gly Ser Gly Ala Gly Ala Gly Ser Gly Ala Gly
705             710                 715                 720

Ala Gly Ser Gly Ala Gly Ala Gly Ser Gly Ala Gly Ala Gly Ser Gly
                725             730                 735

Ala Gly Ala Gly Ser Gly Ala Gly Ala Gly Ser Gly Ala Gly Ala Gly
        740             745                 750

Ser Gly Ala Gly Ala Gly Ser Gly Ala Gly Ala Gly Ser Gly Ala Gly
        755             760                 765

Ala Gly Ser Gly Ala Gly Ala Gly Ser Gly Ala Gly Ala Gly Ser Gly
        770             775                 780

Ala Gly Ala Gly Ser Gly Ala Gly Ala Gly Ser Gly Ala Gly Ala Gly
785             790                 795                 800

Ser Gly Ala Gly Ala Gly Ser Gly Ala Gly Ala Gly Ser Gly Ala Gly
                805             810                 815

Ala Gly Ser Gly Ala Gly Ala Gly Ser Gly Ala Gly Ala Gly Ser Gly
                820             825                 830

Ala Gly Ala Gly Ser Gly Ala Gly Ala Gly Ser Gly Ala Gly Ala Gly
        835             840                 845

Ser Gly Ala Gly Ala Gly Ser Gly Ala Gly Ala Gly Ser Gly Ala Gly
        850             855                 860

Ala Gly Ser Gly Ala Gly Ala Gly Ser Gly Ala Gly Ala Gly Ser Gly
865                 870                 875                 880

```
Ala Gly Ala Gly Ser Gly Ala Gly Ala Gly Ser Gly Ala Gly Ala Gly
            885                 890                 895

Ser Gly Ala Gly Ala Gly Ser Gly Ala Gly Ala Gly Ser Gly Ala Gly
            900                 905                 910

Ala Gly Ser Gly Ala Gly Ala Gly Ser Gly Ala Gly Ala Gly Ser Gly
            915                 920                 925

Ala Gly Ala Gly Ser Gly Ala Gly Ala Gly Ser Gly Ala Gly Ala Gly
        930                 935                 940

Ser Gly Ala Gly Ala Gly Ser Gly Ala Gly Ala Gly Ser Gly Ala Gly
    945                 950                 955                 960

Ala Gly Ser Gly Ala Gly Ala Gly Ser Gly Ala Gly Ala Gly Ser Gly
            965                 970                 975

Ala Gly Ala Gly Ser Gly Ala Gly Ala Gly Ser Gly Ala Gly Ala Gly
            980                 985                 990

Ser Gly Ala Gly Ala Gly Ser Gly  Ala Gly Ala Gly Ser  Gly Ala Gly
        995                 1000                1005

Ala Gly  Ser Gly Ala Gly Ala  Gly Ser Gly Ala Gly  Ala Gly Ser
    1010                1015                1020

Gly Ala  Gly Ala Gly Ser Gly  Ala Gly Ala Gly Ser  Gly Ala Gly
    1025                1030                1035

Ala Gly  Ser Gly Ala Gly Ala  Gly Ser Gly Ala Gly  Ala Gly Ser
    1040                1045                1050

Gly Ala  Gly Ala Gly Ser Gly  Ala Gly Ala Gly Ser  Gly Ala Gly
    1055                1060                1065

Ala Gly  Ser Gly Ala Gly Ala  Gly Ser Met Asp Pro  Gly Arg Tyr
    1070                1075                1080

Gln Asp  Leu Arg Ser His His  His His His His
    1085                1090
```

<210> 20
<211> 830
<212> PRT
<213> Artificial Sequence

<220>

<223> \202b\202k\202o\202R\201D\202V polymer

<400> 20

```
Met Asp Pro Val Val Leu Gln Arg Arg Asp Trp Glu Asn Pro Gly Val
1               5               10              15

Thr Gln Leu Asn Arg Leu Ala Ala His Pro Pro Phe Ala Ser Asp Pro
        20              25              30

Met Gly Ala Pro Gly Thr Pro Gly Pro Gln Gly Leu Pro Gly Ser Pro
        35              40              45

Gly Ala Pro Gly Thr Pro Gly Pro Gln Gly Leu Pro Gly Ser Pro Gly
    50              55              60

Ala Pro Gly Thr Pro Gly Pro Gln Gly Leu Pro Gly Ser Pro Gly Ala
65              70              75              80

Pro Gly Thr Pro Gly Pro Gln Gly Leu Pro Gly Ser Pro Gly Ala Pro
            85              90              95

Gly Thr Pro Gly Pro Gln Gly Leu Pro Gly Ser Pro Gly Ala Pro Gly
        100             105             110

Thr Pro Gly Pro Gln Gly Leu Pro Gly Ser Pro Gly Ala Pro Gly Thr
    115             120             125

Pro Gly Pro Gln Gly Leu Pro Gly Ser Pro Gly Ala Pro Gly Thr Pro
    130             135             140

Gly Pro Gln Gly Leu Pro Gly Ser Pro Gly Ala Pro Gly Thr Pro Gly
145             150             155             160

Pro Gln Gly Leu Pro Gly Ser Pro Gly Ala Pro Gly Thr Pro Gly Pro
            165             170             175

Gln Gly Leu Pro Gly Ser Pro Gly Ala Pro Gly Thr Pro Gly Pro Gln
            180             185             190

Gly Leu Pro Gly Ser Pro Gly Ala Pro Gly Thr Pro Gly Pro Gln Gly
            195             200             205

Leu Pro Gly Ser Pro Gly Ala Pro Gly Thr Pro Gly Pro Gln Gly Leu
    210             215             220

Pro Gly Ser Pro Gly Ala Pro Gly Thr Pro Gly Pro Gln Gly Leu Pro
225             230             235             240
```

```
Gly Ser Pro Gly Ala Pro Gly Thr Pro Gly Pro Gln Gly Leu Pro Gly
              245              250              255

Ser Pro Gly Ala Pro Gly Thr Pro Gly Pro Gln Gly Leu Pro Gly Ser
              260              265              270

Pro Gly Ala Pro Gly Thr Pro Gly Pro Gln Gly Leu Pro Gly Ser Pro
              275              280              285

Gly Ala Pro Gly Thr Pro Gly Pro Gln Gly Leu Pro Gly Ser Pro Gly
    290              295              300

Ala Pro Gly Thr Pro Gly Pro Gln Gly Leu Pro Gly Ser Pro Gly Ala
305              310              315              320

Pro Gly Thr Pro Gly Pro Gln Gly Leu Pro Gly Ser Pro Gly Ala Pro
              325              330              335

Gly Thr Pro Gly Pro Gln Gly Leu Pro Gly Ser Pro Gly Ala Pro Gly
              340              345              350

Thr Pro Gly Pro Gln Gly Leu Pro Gly Ser Pro Gly Ala Pro Gly Thr
    355              360              365

Pro Gly Pro Gln Gly Leu Pro Gly Ser Pro Gly Ala Pro Gly Thr Pro
    370              375              380

Gly Pro Gln Gly Leu Pro Gly Ser Pro Gly Ala Pro Gly Thr Pro Gly
385              390              395              400

Pro Gln Gly Leu Pro Gly Ser Pro Gly Ala Pro Gly Thr Pro Gly Pro
              405              410              415

Gln Gly Leu Pro Gly Ser Pro Gly Ala Pro Gly Thr Pro Gly Pro Gln
              420              425              430

Gly Leu Pro Gly Ser Pro Gly Ala Pro Gly Thr Pro Gly Pro Gln Gly
         435              440              445

Leu Pro Gly Ser Pro Gly Ala Pro Gly Thr Pro Gly Pro Gln Gly Leu
    450              455              460

Pro Gly Ser Pro Gly Ala Pro Gly Thr Pro Gly Pro Gln Gly Leu Pro
465              470              475              480

Gly Ser Pro Gly Ala Pro Gly Thr Pro Gly Pro Gln Gly Leu Pro Gly
              485              490              495
```

Ser Pro Gly Ala Pro Gly Thr Pro Gly Pro Gln Gly Leu Pro Gly Ser
500 505 510

Pro Gly Ala Pro Gly Thr Pro Gly Pro Gln Gly Leu Pro Gly Ser Pro
515 520 525

Gly Ala Pro Gly Thr Pro Gly Pro Gln Gly Leu Pro Gly Ser Pro Gly
530 535 540

Ala Pro Gly Thr Pro Gly Pro Gln Gly Leu Pro Gly Ser Pro Gly Ala
545 550 555 560

Pro Gly Thr Pro Gly Pro Gln Gly Leu Pro Gly Ser Pro Gly Ala Pro
565 570 575

Gly Thr Pro Gly Pro Gln Gly Leu Pro Gly Ser Pro Gly Ala Pro Gly
580 585 590

Thr Pro Gly Pro Gln Gly Leu Pro Gly Ser Pro Gly Ala Pro Gly Thr
595 600 605

Pro Gly Pro Gln Gly Leu Pro Gly Ser Pro Gly Ala Pro Gly Thr Pro
610 615 620

Gly Pro Gln Gly Leu Pro Gly Ser Pro Gly Ala Pro Gly Thr Pro Gly
625 630 635 640

Pro Gln Gly Leu Pro Gly Ser Pro Gly Ala Pro Gly Thr Pro Gly Pro
645 650 655

Gln Gly Leu Pro Gly Ser Pro Gly Ala Pro Gly Thr Pro Gly Pro Gln
660 665 670

Gly Leu Pro Gly Ser Pro Gly Ala Pro Gly Thr Pro Gly Pro Gln Gly
675 680 685

Leu Pro Gly Ser Pro Gly Ala Pro Gly Thr Pro Gly Pro Gln Gly Leu
690 695 700

Pro Gly Ser Pro Gly Ala Pro Gly Thr Pro Gly Pro Gln Gly Leu Pro
705 710 715 720

Gly Ser Pro Gly Ala Pro Gly Thr Pro Gly Pro Gln Gly Leu Pro Gly
725 730 735

Ser Pro Gly Ala Pro Gly Thr Pro Gly Pro Gln Gly Leu Pro Gly Ser

```
                    740                     745                     750


        Pro Gly Ala Pro Gly Thr Pro Gly Pro Gln Gly Leu Pro Gly Ser Pro
                    755                     760                     765



        Gly Ala Pro Gly Thr Pro Gly Pro Gln Gly Leu Pro Gly Ser Pro Gly
                    770                     775                     780



        Ala Pro Gly Thr Pro Gly Pro Gln Gly Leu Pro Gly Ser Pro Gly Ala
        785                     790                     795                     800



        Pro Gly Thr Pro Gly Pro Gln Gly Leu Pro Gly Ser Pro Met Asp Pro
                            805                     810                     815



        Gly Arg Tyr Gln Asp Leu Arg Ser His His His His His His
                            820                     825                     830
```

<210> 21
<211> 850
<212> PRT
<213> Artificial Sequence

<220>
<223> \202d\202k\202o\202P polymer

<400> 21

```
Met Asp Pro Val Val Leu Gln Arg Arg Asp Trp Glu Asn Pro Gly Val
1               5               10              15

Thr Gln Leu Asn Arg Leu Ala Ala His Pro Pro Phe Ala Ser Asp Pro
        20              25              30

Met Val Pro Gly Val Gly Val Pro Gly Val Gly Val Pro Gly Val Gly
        35              40              45

Val Pro Gly Val Gly Val Pro Gly Val Gly Val Pro Gly Val Gly Val
    50              55              60

Pro Gly Val Gly Val Pro Gly Val Gly Val Pro Gly Val Gly Val Pro
65              70              75              80

Gly Val Gly Val Pro Gly Val Gly Val Pro Gly Val Gly Val Pro Gly
                85              90              95

Val Gly Val Pro Gly Val Gly Val Pro Gly Val Gly Val Pro Gly Val
        100             105             110

Gly Val Pro Gly Val Gly Val Pro Gly Val Gly Val Pro Gly Val Gly
        115             120             125
```

```
Val Pro Gly Val Gly Val Pro Gly Val Gly Val Pro Gly Val Gly Val
    130             135             140

Pro Gly Val Gly Val Pro Gly Val Gly Val Pro Gly Val Gly Val Pro
145             150             155             160

Gly Val Gly Val Pro Gly Val Gly Val Pro Gly Val Gly Val Pro Gly
                165             170             175

Val Gly Val Pro Gly Val Gly Val Pro Gly Val Gly Val Pro Gly Val
            180             185             190

Gly Val Pro Gly Val Gly Val Pro Gly Val Gly Val Pro Gly Val Gly
        195             200             205

Val Pro Gly Val Gly Val Pro Gly Val Gly Val Pro Gly Val Gly Val
    210             215             220

Pro Gly Val Gly Val Pro Gly Val Gly Val Pro Gly Val Gly Val Pro
225             230             235             240

Gly Val Gly Val Pro Gly Val Gly Val Pro Gly Val Gly Val Pro Gly
                245             250             255

Val Gly Val Pro Gly Val Gly Val Pro Gly Val Gly Val Pro Gly Val
            260             265             270

Gly Val Pro Gly Val Gly Val Pro Gly Val Gly Val Pro Gly Val Gly
        275             280             285

Val Pro Gly Val Gly Val Pro Gly Val Gly Val Pro Gly Val Gly Val
    290             295             300

Pro Gly Val Gly Val Pro Gly Val Gly Val Pro Gly Val Gly Val Pro
305             310             315             320

Gly Val Gly Val Pro Gly Val Gly Val Pro Gly Val Gly Val Pro Gly
                325             330             335

Val Gly Val Pro Gly Val Gly Val Pro Gly Val Gly Val Pro Gly Val
            340             345             350

Gly Val Pro Gly Val Gly Val Pro Gly Val Gly Val Pro Gly Val Gly
        355             360             365

Val Pro Gly Val Gly Val Pro Gly Val Gly Val Pro Gly Val Gly Val
```

370 375 380

Pro Gly Val Gly Val Pro Gly Val Gly Val Pro Gly Val Gly Val Pro
385 390 395 400

Gly Val Gly Val Pro Gly Val Gly Val Pro Gly Val Gly Val Pro Gly
405 410 415

Val Gly Val Pro Gly Val Gly Val Pro Gly Val Gly Val Pro Gly Val
420 425 430

Gly Val Pro Gly Val Gly Val Pro Gly Val Gly Val Pro Gly Val Gly
435 440 445

Val Pro Gly Val Gly Val Pro Gly Val Gly Val Pro Gly Val Gly Val
450 455 460

Pro Gly Val Gly Val Pro Gly Val Gly Val Pro Gly Val Gly Val Pro
465 470 475 480

Gly Val Gly Val Pro Gly Val Gly Val Pro Gly Val Gly Val Pro Gly
485 490 495

Val Gly Val Pro Gly Val Gly Val Pro Gly Val Gly Val Pro Gly Val
500 505 510

Gly Val Pro Gly Val Gly Val Pro Gly Val Gly Val Pro Gly Val Gly
515 520 525

Val Pro Gly Val Gly Val Pro Gly Val Gly Val Pro Gly Val Gly Val
530 535 540

Pro Gly Val Gly Val Pro Gly Val Gly Val Pro Gly Val Gly Val Pro
545 550 555 560

Gly Val Gly Val Pro Gly Val Gly Val Pro Gly Val Gly Val Pro Gly
565 570 575

Val Gly Val Pro Gly Val Gly Val Pro Gly Val Gly Val Pro Gly Val
580 585 590

Gly Val Pro Gly Val Gly Val Pro Gly Val Gly Val Pro Gly Val Gly
595 600 605

Val Pro Gly Val Gly Val Pro Gly Val Gly Val Pro Gly Val Gly Val
610 615 620

```
Pro Gly Val Gly Val Pro Gly Val Gly Val Pro Gly Val Gly Val Pro
625                 630                 635                 640

Gly Val Gly Val Pro Gly Val Gly Val Pro Gly Val Gly Val Pro Gly
                645                 650                 655

Val Gly Val Pro Gly Val Gly Val Pro Gly Val Gly Val Pro Gly Val
            660                 665                 670

Gly Val Pro Gly Val Gly Val Pro Gly Val Gly Val Pro Gly Val Gly
            675                 680                 685

Val Pro Gly Val Gly Val Pro Gly Val Gly Val Pro Gly Val Gly Val
    690                 695                 700

Pro Gly Val Gly Val Pro Gly Val Gly Val Pro Gly Val Gly Val Pro
705                 710                 715                 720

Gly Val Gly Val Pro Gly Val Gly Val Pro Gly Val Gly Val Pro Gly
                725                 730                 735

Val Gly Val Pro Gly Val Gly Val Pro Gly Val Gly Val Pro Gly Val
            740                 745                 750

Gly Val Pro Gly Val Gly Val Pro Gly Val Gly Val Pro Gly Val Gly
            755                 760                 765

Val Pro Gly Val Gly Val Pro Gly Val Gly Val Pro Gly Val Gly Val
    770                 775                 780

Pro Gly Val Gly Val Pro Gly Val Gly Val Pro Gly Val Gly Val Pro
785                 790                 795                 800

Gly Val Gly Val Pro Gly Val Gly Val Pro Gly Val Gly Val Pro Gly
                805                 810                 815

Val Gly Val Pro Gly Val Gly Val Pro Gly Val Gly Val Pro Gly Val
            820                 825                 830

Gly Met Asp Pro Gly Arg Tyr Gln Asp Leu Arg Ser His His His His
        835                 840                 845

His His
    850
```

<210> 22
<211> 1058

63

<212> PRT
<213> Artificial Sequence

<220>
<223> \202c\202b\202o\202Q polymer

<400> 22

```
Met Asp Pro Val Val Leu Gln Arg Arg Asp Trp Glu Asn Pro Gly Val
1               5                   10                  15

Thr Gln Leu Asn Arg Leu Ala Ala His Pro Pro Phe Ala Ser Asp Pro
            20                  25                  30

Met Gly Ala His Gly Pro Ala Gly Pro Lys Gly Ala His Gly Pro Ala
        35              40                  45

Gly Pro Lys Gly Ala Pro Gly Pro Ala Gly Pro Pro Gly Ala Pro Gly
    50              55                  60

Pro Ala Gly Pro Pro Gly Ala Pro Gly Pro Ala Gly Pro Pro Gly Ala
65                  70              75                  80

Pro Gly Pro Ala Gly Pro Pro Gly Ala Pro Gly Pro Ala Gly Pro Pro
                85                  90                  95

Gly Ala Pro Gly Pro Ala Gly Pro Pro Gly Ala Pro Gly Pro Ala Gly
            100             105             110

Pro Pro Gly Ala Pro Gly Pro Ala Gly Pro Pro Gly Ala Pro Gly Pro
        115             120                 125

Ala Gly Pro Pro Gly Ala Pro Gly Pro Ala Gly Pro Pro Gly Ala Pro
    130             135                 140

Gly Pro Ala Gly Pro Pro Gly Ala Pro Gly Pro Ala Gly Pro Pro Gly
145                 150                 155                 160

Ala His Gly Pro Ala Gly Pro Lys Gly Ala His Gly Pro Ala Gly Pro
                165                 170                 175

Lys Gly Ala His Gly Pro Ala Gly Pro Lys Gly Ala His Gly Pro Ala
        180             185                 190

Gly Pro Lys Gly Ala Pro Gly Pro Ala Gly Pro Pro Gly Ala Pro Gly
        195             200                 205

Pro Ala Gly Pro Pro Gly Ala Pro Gly Pro Ala Gly Pro Pro Gly Ala
210                 215                 220
```

65

```
Pro Gly Pro Ala Gly Pro Pro Gly Ala Pro Gly Pro Ala Gly Pro Pro
225             230             235             240

Gly Ala Pro Gly Pro Ala Gly Pro Pro Gly Ala Pro Gly Pro Ala Gly
                245             250             255

Pro Pro Gly Ala Pro Gly Pro Ala Gly Pro Pro Gly Ala Pro Gly Pro
            260             265             270

Ala Gly Pro Pro Gly Ala Pro Gly Pro Ala Gly Pro Pro Gly Ala Pro
            275             280             285

Gly Pro Ala Gly Pro Pro Gly Ala Pro Gly Pro Ala Gly Pro Pro Gly
        290             295             300

Ala His Gly Pro Ala Gly Pro Lys Gly Ala His Gly Pro Ala Gly Pro
305             310             315             320

Lys Gly Ala His Gly Pro Ala Gly Pro Lys Gly Ala His Gly Pro Ala
                325             330             335

Gly Pro Lys Gly Ala Pro Gly Pro Ala Gly Pro Pro Gly Ala Pro Gly
        340             345             350

Pro Ala Gly Pro Pro Gly Ala Pro Gly Pro Ala Gly Pro Pro Gly Ala
        355             360             365

Pro Gly Pro Ala Gly Pro Pro Gly Ala Pro Gly Pro Ala Gly Pro Pro
    370             375             380

Gly Ala Pro Gly Pro Ala Gly Pro Pro Gly Ala Pro Gly Pro Ala Gly
385             390             395             400

Pro Pro Gly Ala Pro Gly Pro Ala Gly Pro Pro Gly Ala Pro Gly Pro
            405             410             415

Ala Gly Pro Pro Gly Ala Pro Gly Pro Ala Gly Pro Pro Gly Ala Pro
            420             425             430

Gly Pro Ala Gly Pro Pro Gly Ala Pro Gly Pro Ala Gly Pro Pro Gly
        435             440             445

Ala His Gly Pro Ala Gly Pro Lys Gly Ala His Gly Pro Ala Gly Pro
        450             455             460

Lys Gly Ala His Gly Pro Ala Gly Pro Lys Gly Ala His Gly Pro Ala
465             470             475             480
```

Gly Pro Lys Gly Ala Pro Gly Pro Ala Gly Pro Pro Gly Ala Pro Gly
485 490 495

Pro Ala Gly Pro Pro Gly Ala Pro Gly Pro Ala Gly Pro Pro Gly Ala
500 505 510

Pro Gly Pro Ala Gly Pro Pro Gly Ala Pro Gly Pro Ala Gly Pro Pro
515 520 525

Gly Ala Pro Gly Pro Ala Gly Pro Pro Gly Ala Pro Gly Pro Ala Gly
530 535 540

Pro Pro Gly Ala Pro Gly Pro Ala Gly Pro Pro Gly Ala Pro Gly Pro
545 550 555 560

Ala Gly Pro Pro Gly Ala Pro Gly Pro Ala Gly Pro Pro Gly Ala Pro
565 570 575

Gly Pro Ala Gly Pro Pro Gly Ala Pro Gly Pro Ala Gly Pro Pro Gly
580 585 590

Ala His Gly Pro Ala Gly Pro Lys Gly Ala His Gly Pro Ala Gly Pro
595 600 605

Lys Gly Ala His Gly Pro Ala Gly Pro Lys Gly Ala His Gly Pro Ala
610 615 620

Gly Pro Lys Gly Ala Pro Gly Pro Ala Gly Pro Pro Gly Ala Pro Gly
625 630 635 640

Pro Ala Gly Pro Pro Gly Ala Pro Gly Pro Ala Gly Pro Pro Gly Ala
645 650 655

Pro Gly Pro Ala Gly Pro Pro Gly Ala Pro Gly Pro Ala Gly Pro Pro
660 665 670

Gly Ala Pro Gly Pro Ala Gly Pro Pro Gly Ala Pro Gly Pro Ala Gly
675 680 685

Pro Pro Gly Ala Pro Gly Pro Ala Gly Pro Pro Gly Ala Pro Gly Pro
690 695 700

Ala Gly Pro Pro Gly Ala Pro Gly Pro Ala Gly Pro Pro Gly Ala Pro
705 710 715 720

Gly Pro Ala Gly Pro Pro Gly Ala Pro Gly Pro Ala Gly Pro Pro Gly
725 730 735

```
Ala His Gly Pro Ala Gly Pro Lys Gly Ala His Gly Pro Ala Gly Pro
            740             745             750

Lys Gly Ala His Gly Pro Ala Gly Pro Lys Gly Ala His Gly Pro Ala
        755             760             765

Gly Pro Lys Gly Ala Pro Gly Pro Ala Gly Pro Pro Gly Ala Pro Gly
        770             775             780

Pro Ala Gly Pro Pro Gly Ala Pro Gly Pro Ala Gly Pro Pro Gly Ala
785             790             795             800

Pro Gly Pro Ala Gly Pro Pro Gly Ala Pro Gly Pro Ala Gly Pro Pro
            805             810             815

Gly Ala Pro Gly Pro Ala Gly Pro Pro Gly Ala Pro Gly Pro Ala Gly
            820             825             830

Pro Pro Gly Ala Pro Gly Pro Ala Gly Pro Pro Gly Ala Pro Gly Pro
            835             840             845

Ala Gly Pro Pro Gly Ala Pro Gly Pro Ala Gly Pro Pro Gly Ala Pro
    850             855             860

Gly Pro Ala Gly Pro Pro Gly Ala Pro Gly Pro Ala Gly Pro Pro Gly
865             870             875             880

Ala His Gly Pro Ala Gly Pro Lys Gly Ala His Gly Pro Ala Gly Pro
            885             890             895

Lys Gly Ala His Gly Pro Ala Gly Pro Lys Gly Ala His Gly Pro Ala
        900             905             910

Gly Pro Lys Gly Ala Pro Gly Pro Ala Gly Pro Pro Gly Ala Pro Gly
        915             920             925

Pro Ala Gly Pro Pro Gly Ala Pro Gly Pro Ala Gly Pro Pro Gly Ala
    930             935             940

Pro Gly Pro Ala Gly Pro Pro Gly Ala Pro Gly Pro Ala Gly Pro Pro
945             950             955             960

Gly Ala Pro Gly Pro Ala Gly Pro Pro Gly Ala Pro Gly Pro Ala Gly
            965             970             975

Pro Pro Gly Ala Pro Gly Pro Ala Gly Pro Pro Gly Ala Pro Gly Pro
```

```
                 980                    985                         990


        Ala Gly Pro Pro Gly Ala Pro Gly  Pro Ala Gly Pro Pro  Gly Ala Pro
                995                  1000                   1005


        Gly Pro  Ala Gly Pro Pro Gly  Ala Pro Gly Pro Ala  Gly Pro Pro
            1010            1015                   1020


        Gly Ala  His Gly Pro Ala Gly  Pro Lys Gly Ala His  Gly Pro Ala
            1025            1030                   1035


        Gly Pro  Lys Met Asp Pro Gly  Arg Tyr Gln Asp Leu  Arg Ser His
            1040            1045                   1050


        His His  His His His
            1055
```

<210> 23
<211> 1110
<212> PRT
<213> Artificial Sequence

<220>
<223> \202r\202k\202o\202S\201D\202Q polymer

<400> 23

```
Met Asp Pro Val Val Leu Gln Arg Arg Asp Trp Glu Asn Pro Gly Val
1               5                   10                  15

Thr Gln Leu Asn Arg Leu Ala Ala His Pro Pro Phe Ala Ser Asp Pro
            20                  25                  30

Met Gly Ala Gly Ala Gly Ser Gly Ala Gly Ala Gly Ser Gly Ala Gly
        35                  40                  45

Ala Gly Ser Gly Ala Gly Ala Gly Ser Gly Ala Gly Ala Gly Ser Gly
    50                  55                  60

Ala Gly Ala Gly Ser Gly Lys Gly Val Pro Gly Lys Gly Val Pro Gly
65                  70                  75                  80

Ala Gly Ala Gly Ser Gly Ala Gly Ala Gly Ser Gly Ala Gly Ala Gly
                85                  90                  95

Ser Gly Ala Gly Ala Gly Ser Gly Ala Gly Ala Gly Ser Gly Ala Gly
            100                 105                 110

Ala Gly Ser Gly Ala Gly Ala Gly Ser Gly Ala Gly Ala Gly Ser Gly
        115                 120                 125
```

```
Ala Gly Ala Gly Ser Gly Ala Gly Ala Gly Ser Gly Ala Gly Ala Gly
    130                 135                 140

Ser Gly Ala Gly Ala Gly Ser Gly Ala Gly Ala Gly Ser Gly Ala Gly
145                 150                 155                 160

Ala Gly Ser Gly Ala Gly Ala Gly Ser Gly Ala Gly Ala Gly Ser Gly
                165                 170                 175

Lys Gly Val Pro Gly Lys Gly Val Pro Gly Ala Gly Ala Gly Ser Gly
            180                 185                 190

Ala Gly Ala Gly Ser Gly Ala Gly Ala Gly Ser Gly Ala Gly Ala Gly
        195                 200                 205

Ser Gly Ala Gly Ala Gly Ser Gly Ala Gly Ala Gly Ser Gly Ala Gly
    210                 215                 220

Ala Gly Ser Gly Ala Gly Ala Gly Ser Gly Ala Gly Ala Gly Ser Gly
225                 230                 235                 240

Ala Gly Ala Gly Ser Gly Ala Gly Ala Gly Ser Gly Ala Gly Ala Gly
                245                 250                 255

Ser Gly Ala Gly Ala Gly Ser Gly Ala Gly Ala Gly Ser Gly Ala Gly
        260                 265                 270

Ala Gly Ser Gly Ala Gly Ala Gly Ser Gly Lys Gly Val Pro Gly Lys
        275                 280                 285

Gly Val Pro Gly Ala Gly Ala Gly Ser Gly Ala Gly Ala Gly Ser Gly
    290                 295                 300

Ala Gly Ala Gly Ser Gly Ala Gly Ala Gly Ser Gly Ala Gly Ala Gly
305                 310                 315                 320

Ser Gly Ala Gly Ala Gly Ser Gly Ala Gly Ala Gly Ser Gly Ala Gly
                325                 330                 335

Ala Gly Ser Gly Ala Gly Ala Gly Ser Gly Ala Gly Ala Gly Ser Gly
        340                 345                 350

Ala Gly Ala Gly Ser Gly Ala Gly Ala Gly Ser Gly Ala Gly Ala Gly
    355                 360                 365

Ser Gly Ala Gly Ala Gly Ser Gly Ala Gly Ala Gly Ser Gly Ala Gly
```

```
          370                      375                      380

     Ala Gly Ser Gly Lys Gly Val Pro Gly Lys Gly Val Pro Gly Ala Gly
     385             390             395                      400


     Ala Gly Ser Gly Ala Gly Ala Gly Ser Gly Ala Gly Ala Gly Ser Gly
                 405             410                      415


     Ala Gly Ala Gly Ser Gly Ala Gly Ala Gly Ser Gly Ala Gly Ala Gly
             420             425                      430


     Ser Gly Ala Gly Ala Gly Ser Gly Ala Gly Ala Gly Ser Gly Ala Gly
             435             440                      445


     Ala Gly Ser Gly Ala Gly Ala Gly Ser Gly Ala Gly Ala Gly Ser Gly
             450             455                      460


     Ala Gly Ala Gly Ser Gly Ala Gly Ala Gly Ser Gly Ala Gly Ala Gly
     465             470             475                      480


     Ser Gly Ala Gly Ala Gly Ser Gly Ala Gly Ala Gly Ser Gly Lys Gly
                 485             490                      495


     Val Pro Gly Lys Gly Val Pro Gly Ala Gly Ala Gly Ser Gly Ala Gly
                 500             505                      510


     Ala Gly Ser Gly Ala Gly Ala Gly Ser Gly Ala Gly Ala Gly Ser Gly
                 515             520                      525


     Ala Gly Ala Gly Ser Gly Ala Gly Ala Gly Ser Gly Ala Gly Ala Gly
             530             535                      540


     Ser Gly Ala Gly Ala Gly Ser Gly Ala Gly Ala Gly Ser Gly Ala Gly
     545             550             555                      560


     Ala Gly Ser Gly Ala Gly Ala Gly Ser Gly Ala Gly Ala Gly Ser Gly
                 565             570                      575


     Ala Gly Ala Gly Ser Gly Ala Gly Ala Gly Ser Gly Ala Gly Ala Gly
             580             585                      590


     Ser Gly Ala Gly Ala Gly Ser Gly Lys Gly Val Pro Gly Lys Gly Val
             595             600                      605


     Pro Gly Ala Gly Ala Gly Ser Gly Ala Gly Ala Gly Ser Gly Ala Gly
             610             615                      620
```

```
Ala Gly Ser Gly Ala Gly Ala Gly Ser Gly Ala Gly Ala Gly Ser Gly
625             630         635             640

Ala Gly Ala Gly Ser Gly Ala Gly Ala Gly Ser Gly Ala Gly Ala Gly
                645         650             655

Ser Gly Ala Gly Ala Gly Ser Gly Ala Gly Ala Gly Ser Gly Ala Gly
        660         665         670

Ala Gly Ser Gly Ala Gly Ala Gly Ser Gly Ala Gly Ala Gly Ser Gly
        675         680             685

Ala Gly Ala Gly Ser Gly Ala Gly Ala Gly Ser Gly Ala Gly Ala Gly
    690         695             700

Ser Gly Lys Gly Val Pro Gly Lys Gly Val Pro Gly Ala Gly Ala Gly
705             710             715             720

Ser Gly Ala Gly Ala Gly Ser Gly Ala Gly Ala Gly Ser Gly Ala Gly
            725             730             735

Ala Gly Ser Gly Ala Gly Ala Gly Ser Gly Ala Gly Ala Gly Ser Gly
        740             745             750

Ala Gly Ala Gly Ser Gly Ala Gly Ala Gly Ser Gly Ala Gly Ala Gly
        755             760             765

Ser Gly Ala Gly Ala Gly Ser Gly Ala Gly Ala Gly Ser Gly Ala Gly
    770             775             780

Ala Gly Ser Gly Ala Gly Ala Gly Ser Gly Ala Gly Ala Gly Ser Gly
785             790             795             800

Ala Gly Ala Gly Ser Gly Ala Gly Ala Gly Ser Gly Lys Gly Val Pro
            805             810             815

Gly Lys Gly Val Pro Gly Ala Gly Ala Gly Ser Gly Ala Gly Ala Gly
        820             825             830

Ser Gly Ala Gly Ala Gly Ser Gly Ala Gly Ala Gly Ser Gly Ala Gly
        835             840             845

Ala Gly Ser Gly Ala Gly Ala Gly Ser Gly Ala Gly Ala Gly Ser Gly
    850             855             860

Ala Gly Ala Gly Ser Gly Ala Gly Ala Gly Ser Gly Ala Gly Ala Gly
865             870             875             880
```

```
Ser Gly Ala Gly Ala Gly Ser Gly Ala Gly Ala Gly Ser Gly Ala Gly
            885                 890                 895

Ala Gly Ser Gly Ala Gly Ala Gly Ser Gly Ala Gly Ala Gly Ser Gly
            900                 905                 910

Ala Gly Ala Gly Ser Gly Lys Gly Val Pro Gly Lys Gly Val Pro Gly
            915                 920                 925

Ala Gly Ala Gly Ser Gly Ala Gly Ala Gly Ser Gly Ala Gly Ala Gly
    930                 935                 940

Ser Gly Ala Gly Ala Gly Ser Gly Ala Gly Ala Gly Ser Gly Ala Gly
945                 950                 955                 960

Ala Gly Ser Gly Ala Gly Ala Gly Ser Gly Ala Gly Ala Gly Ser Gly
            965                 970                 975

Ala Gly Ala Gly Ser Gly Ala Gly Ala Gly Ser Gly Ala Gly Ala Gly
            980                 985                 990

Ser Gly Ala Gly Ala Gly Ser Gly  Ala Gly Ala Gly Ser  Gly Ala Gly
    995                 1000                1005

Ala Gly  Ser Gly Ala Gly Ala  Gly Ser Gly Ala Gly  Ala Gly Ser
    1010                1015                1020

Gly Lys  Gly Val Pro Gly Lys  Gly Val Pro Gly Ala  Gly Ala Gly
    1025                1030                1035

Ser Gly  Ala Gly Ala Gly Ser  Gly Ala Gly Ala Gly  Ser Gly Ala
    1040                1045                1050

Gly Ala  Gly Ser Gly Ala Gly  Ala Gly Ser Gly Ala  Gly Ala Gly
    1055                1060                1065

Ser Gly  Ala Gly Ala Gly Ser  Gly Ala Gly Ala Gly  Ser Gly Ala
    1070                1075                1080

Gly Ala  Gly Ser Gly Ala Gly  Ala Gly Ser Met Asp  Pro Gly Arg
    1085                1090                1095

Tyr Gln  Asp Leu Arg Ser His  His His His His His
    1100                1105                1110
```

<210> 24
<211> 490

<212> PRT
<213> Artificial Sequence

<220>
<223> \202d\202k\202o\202P\201D\202Q polymer

<400> 24

```
Met Asp Pro Val Val Leu Gln Arg Arg Asp Trp Glu Asn Pro Gly Val
1               5                   10                  15


Thr Gln Leu Asn Arg Leu Ala Ala His Pro Pro Phe Ala Ser Asp Pro
            20                  25                  30


Met Val Pro Gly Val Gly Val Pro Gly Val Gly Val Pro Gly Val Gly
        35                  40                  45


Val Pro Gly Val Gly Val Val Val Pro Gly Val Gly Val Pro Gly Val
    50                  55                  60


Gly Val Pro Gly Val Gly Val Pro Gly Val Gly Val Val Val Pro Gly
65                  70                  75                  80


Val Gly Val Pro Gly Val Gly Val Pro Gly Val Gly Val Pro Gly Val
                85                  90                  95


Gly Val Val Val Pro Gly Val Gly Val Pro Gly Val Gly Val Pro Gly
            100                 105                 110


Val Gly Val Pro Gly Val Gly Val Val Val Pro Gly Val Gly Val Pro
        115                 120                 125


Gly Val Gly Val Pro Gly Val Gly Val Pro Gly Val Gly Val Val Val
    130                 135                 140


Pro Gly Val Gly Val Pro Gly Val Gly Val Pro Gly Val Gly Val Pro
145                 150                 155                 160


Gly Val Gly Val Val Val Pro Gly Val Gly Val Pro Gly Val Gly Val
            165                 170                 175


Pro Gly Val Gly Val Pro Gly Val Gly Val Val Val Pro Gly Val Gly
        180                 185                 190


Val Pro Gly Val Gly Val Pro Gly Val Gly Val Pro Gly Val Gly Val
    195                 200                 205


Val Val Pro Gly Val Gly Val Pro Gly Val Gly Val Pro Gly Val Gly
    210                 215                 220
```

```
Val Pro Gly Val Gly Val Val Val Pro Gly Val Gly Val Pro Gly Val
225             230             235             240

Gly Val Pro Gly Val Gly Val Pro Gly Val Gly Val Val Val Pro Gly
            245             250             255

Val Gly Val Pro Gly Val Gly Val Pro Gly Val Gly Val Pro Gly Val
            260             265             270

Gly Val Val Val Pro Gly Val Gly Val Pro Gly Val Gly Val Pro Gly
            275             280             285

Val Gly Val Pro Gly Val Gly Val Val Val Pro Gly Val Gly Val Pro
    290             295             300

Gly Val Gly Val Pro Gly Val Gly Val Pro Gly Val Gly Val Val Val
305             310             315             320

Pro Gly Val Gly Val Pro Gly Val Gly Val Pro Gly Val Gly Val Pro
            325             330             335

Gly Val Gly Val Val Val Pro Gly Val Gly Val Pro Gly Val Gly Val
            340             345             350

Pro Gly Val Gly Val Pro Gly Val Gly Val Val Val Pro Gly Val Gly
            355             360             365

Val Pro Gly Val Gly Val Pro Gly Val Gly Val Pro Gly Val Gly Val
    370             375             380

Val Val Pro Gly Val Gly Val Pro Gly Val Gly Val Pro Gly Val Gly
385             390             395             400

Val Pro Gly Val Gly Val Val Val Pro Gly Val Gly Val Pro Gly Val
            405             410             415

Gly Val Pro Gly Val Gly Val Pro Gly Val Gly Val Val Val Pro Gly
            420             425             430

Val Gly Val Pro Gly Val Gly Val Pro Gly Val Gly Val Pro Gly Val
    435             440             445

Gly Val Val Val Pro Gly Val Gly Val Pro Gly Val Gly Val Pro Gly
    450             455             460

Val Gly Val Pro Gly Val Gly Val Val Met Asp Pro Gly Arg Tyr Gln
465             470             475             480
```

77

```
Asp Leu Arg Ser His His His His His His
              485                 490
```